# EUROPEAN PATENT SPECIFICATION

(11) **EP 2 270 512 B1**
(45) Date of publication and mention of the grant of the patent: **23.03.2016**
(21) Application number: 10171757.7
(22) Date of filing: 08.12.2005
(51) Int. Cl.: G01N 33/68

(54) **Methods for diagnosis of Crohn's disease**
Verfahren zur Diagnose von Morbus Crohn
Procédés pour le diagnostic de la maladie de Crohn

(30) Priority: 08.12.2004 US 634339 P
(43) Date of publication of application: 05.01.2011
(62) Divisional of application: 05853294.6
(73) Proprietor: Cedars-Sinai Medical Center, Los Angeles, CA 90048 (US)
(72) Inventor: Targan, Stephan R., Santa Monica, CA 90402 (US); Landers, Carol J., El Segundo, CA 90245 (US)
(74) Representative: Holmes, Michael Anthony

(56) References cited:
- EP-B1- 1 285 271
- WO-A-2004/048600
- TARGAN STEPHAN R ET AL: "Antibodies to a novel flagellin (CBir1) define a unique serologic response in Crohn's disease (CD)" GASTROENTEROLOGY, ELSEVIER, PHILADELPHIA, PA, US, vol. 126, no. 4 Suppl 2, April 2004 (2004-04), page A113, XP009098183 ISSN: 0016-5085
- TARGAN, S. R. ET AL: "Antibodies to CBir1 flagellin define a unique response that is associated independently with complicated Crohn's disease" GASTROENTEROLOGY, vol. 128, 2005, pages 2020-2028, XP002477947

## Description

### FIELD OF INVENTION

This invention relates to methods useful in the medical arts. In particular, various embodiments of the present invention relate to methods for diagnosis and treatment of Crohn's Disease.

### BACKGROUND

The following description includes information that may be useful in understanding the present invention. It is not an admission that any of the information provided herein is prior art or relevant to the presently claimed invention, or that any publication specifically or implicitly referenced is prior art.

Crohn's disease (CD) and ulcerative colitis (UC), collectively referred to as inflammatory bowel disease (IBD), are relatively common inflammatory diseases of the gastrointestinal (GI) tract. Histopathologically and anatomically, these two conditions are distinct, with CD characterized by transmural inflammation that can occur throughout the GI tract, and UC characterized by more superficial inflammation confined to the colon and rectum. Interestingly, both diseases are dependent upon factors present within the complex intestinal microbiota. Indeed, a unifying hypothesis has emerged that proposes that IBD results from a dysregulated mucosal immune response to the intestinal microbiota in genetically susceptible individuals (Strober W, Fuss IJ, Blumberg RS. The immunology of mucosal models of inflammation. Annu. Rev. Immunol. 2002;20:495-549. Bouma G, Strober W. The immunological and genetic basis of inflammatory bowel disease. Nat. Rev. Immunol. 2003;3:521-533.).

While the dependence of IBD on intestinal microbes is increasingly clear, the molecular mechanisms underlying this dependence are not. The intestinal mucosa is exposed to the largest concentration of foreign bacterial antigens of any tissue in the body, estimated to be up to 10¹² organisms per gram of stool in the normal colon. An emerging concept is that there is an active "dialogue" between the microbiota, intestinal epithelial cells, and mucosal immune cells, with each partner communicating with the others (McCracken VJ, Lorenz RG. The gastrointestinal ecosystem: a precarious alliance among epithelium, immunity and microbiota. Cell. Microbiol. 2001;3:1-11.). In this context, "innate" immune responses, which recognize conserved microbial products such as lipopolysaccharide (LPS) and peptidoglycan (PG), are likely to be important in these microbial-host interactions and intestinal homeostasis. Critical to the host's "sensing" of microbes are members of the Toll-like receptor (TLR) family that, alone or in combination, recognize a wide array of microbe-associated molecular patterns on either pathogens or commensals (Kopp E, Medzhitov R. Recognition of microbial infection by Toll-like receptors. Curr. Opin. Immunol. 2003;15:396-401. Akira S. Mammalian Toll-like receptors. Curr. Opin. Immunol. 2003;15:5-11. Sieling PA, Modlin RL. Toll-like receptors: mammalian 'taste receptors' for a smorgasbord of microbial invaders. Current Opin. Microbiol. 2002;5:70-75.). Various TLRs are expressed on intestinal epithelial cells (Cario E, Podolsky DK. Differential alteration in intestinal epithelial cell expression of toll-like receptor 3 (TLR3) and TLR4 in inflammatory bowel disease. Infect. Immunol. 2000;68:7010-7017. Gewirtz AT, Navas TA, Lyons S, Godowski PJ, Madara JL. Cutting Edge: Bacterial flagellin activates basolaterally expressed TLR5 to induce epithelial proinflammatory gene expression. J. Immunol. 2001;167:1882-1885. Abreu MT, et al. TLR4 and MD-2 expression is regulated by immune-mediated signals in human intestinal epithelial cells. J. Biol. Chem. 2002;277:20431-20437. Hershberg RM. The epithelial cell cytoskeleton and intracellular trafficking V. Polarized compartmentalization of antigen processing and Toll-like receptor signaling in intestinal epithelial cells. Am. J. Physiol. Gastrointest. Liver Physiol. 2002;283:G833-G839.) and more broadly on macrophages and dendritic cells in the lamina propria.

Given the involvement of innate immune mechanisms in the modulation of T cell responses, the bacterial dependence of IBD is likely to involve both bacterial products such as LPS, PG, and other TLR ligands, and specific bacterial antigens capable of stimulating CD4⁺ T cell responses. CD4⁺ T lymphocytes have been identified as the crucial effector cells in experimental models of IBD (Berg DJ, et al. Enterocolitis and colon cancer in interleukin-10-deficient mice are associated with aberrant cytokine production and CD4+ TH1-like responses. J. Clin. Invest. 1996;98:1010-1020. Powrie F, et al. Inhibition of Th1 responses prevents inflammatory bowel disease in scid mice reconstituted with CD45RBhi CD4+ T cells. Immunity. 1994;1:553-562. Cong Y, et al. CD4+ T cells reactive to enteric bacterial antigens in spontaneously colitic C3H/HeJBir mice: increased T helper cell Type 1 response and ability to transfer disease. J. Exp. Med. 1998;187:855-864.), and these pathogenic CD4⁺ T cell responses are directed against the enteric microbiota. Enteric bacterial antigen-reactive CD4⁺ T cells are able to induce colitis when adoptively transferred into immunodeficient recipients (Cong Y, et al. CD4+ T cells reactive to enteric bacterial antigens in spontaneously colitic C3H/HeJBir mice: increased T helper cell Type 1 response and ability to transfer disease. J. Exp. Med. 1998;187:855-864.). The in vitro data suggest that there is a relatively small number of immunodominant antigens that stimulate the pathogenic T cell responses (Brandwein SL, et al. Spontaneously colitic C3H/HeJBir mice demonstrate selective antibody reactivity to antigens of the enteric bacterial flora. J. Immunol. 1997;159:44-52), but the complexity of the intestinal microflora has posed a significant challenge to their identification.

In humans, specific associations between particular bacterial species and the development of disease or its characteristics have not been established. Immune responses to commensal enteric organisms have been investigated in CD. It was been shown that CD patients have antibodies to specific bacterial antigens and that patients can be clustered into 4 groups depending on their antibody response patterns (Landers CJ, Cohavy O, Misra R, Yang H, Lin YC, Braun J, Targan SR. Selected loss of tolerance evidenced by Crohn's disease-associated immune responses to auto- and microbial antigens. Gastroenterology 2002;123:689-99.). These clusters are (1) antibody responses against oligomannan (anti-*Saccharomyces cerevisiae*; ASCA), (2) antibody responses to both *Escherichia coli* outer membrane protein C (anti-OmpC) and a CD-related protein from *Pseudomonas fluorescens* (anti-CD-related bacterial sequence {I2}), (3) antibody responses to nuclear antigens (perinuclear antineutrophil cytoplasmic antibody; pANCA), or (4) low or no serological response to any of the tested antigens. These distinct antibody response patterns may indicate unique pathophysiological mechanisms in the progression of this complicated disease. In addition, phenotypic associations with specific serological response patterns have been discovered (Landers CJ, Cohavy O, Misra R, Yang H, Lin YC, Braun J, Targan SR. Selected loss of tolerance evidenced by Crohn's disease-associated immune responses to auto- and microbial antigens. Gastroenterology 2002;123:689-99. Vasiliauskas EA, Plevy SE, Landers CJ, Binder SW, Ferguson DM, Yang H, Rotter JI, Vidrich A, Targan SR. Perinuclear antineutrophil cytoplasmic antibodies in patients with Crohn's disease define a clinical subgroup. Gastroenterology 1996;110:1810-9. Vasiliauskas EA, Kam LY, Karp LC, Gaiennie J, Yang H, Targan SR. Marker antibody expression stratifies Crohn's disease into immunologically homogeneous subgroups with distinct clinical characteristics. Gut 2000;47:487-96. Mow WS, Vasiliauskas EA, Lin YC, Fleshner PR, Papadakis KA, Taylor KD, Landers CJ, Abreu-Martin MT, Rotter JI, Yang H, Targan SR. Association of antibody responses to microbial antigens and complications of small bowel Crohn's disease. Gastroenterology 2004;126:414-24.).

Immunologic responses to bacterial products are key to the induction of inflammatory bowel disease in humans and in experimental models. The relationship of these immune responses to the underlying genetic and clinical phenotypes is just beginning to emerge. Thus, among patients with Crohn's disease, immune responses to different microbial antigens may be related to different pathophysiologic mechanisms, and may represent distinct genotypes and phenotypes.

Thus, there is need in the art to associate clinical phenotypes of Crohn's disease with various antigens, as such determination can enable more appropriate treatments for the disease. Furthermore, there exists a need for the diagnosis and treatment of Crohn's disease and subtypes of Crohn's disease.

### SUMMARY OF INVENTION

The following embodiments and aspects thereof are described and illustrated in conjunction with compositions and methods which are meant to be exemplary and illustrative, not limiting in scope.

Various embodiments of the present invention provide for methods for diagnosing Crohn's disease in a mammal. Additional embodiments provide for determining a subtype of Crohn's disease, such as a phenotypic feature associated with Crohn's disease. Further embodiments provide for treating Crohn's disease. In one embodiment, the mammal is a human.

In particular embodiments, diagnosing Crohn's disease may be performed by determining the presence of anti-CBir1 expression, where the presence of anti-CBir1 expression indicates that the mammal has Crohn's disease. Determining a subtype of Crohn's disease, such as a phenotypic feature associated with Crohn's disease may also be performed by determining the presence of anti-CBir1 expression, where the presence of anti-CBir1 indicates that the mammal has small bowel disease, internal penetrating/perforating disease or fibrostenosing disease.

Determining the presence of anti-CBir1 expression may be accomplished by various techniques. For example, determining the presence of anti-CBir1 expression may be performed by determining the presence of an RNA sequence or a fragment of an RNA sequence that encodes an anti-CBir1 antibody; for example, using Northern blot analysis or reverse transcription-polymerase chain reaction (RT-PCR). Determining the presence of anti-CBir1 expression may also be performed by determining the presence of anti-CBir1 antibodies; for example IgG anti-CBir1. Anti-CBir1 antibodies are not limited to IgG, as IgA, IgM, IgD and IgE are also contemplated in connection with various embodiments of the present invention. These examples are not intended to be limiting, as one skilled in the art will recognize other appropriate means for determining the presence of anti-CBir1 expression.

Determining the presence of anti-CBir1 antibodies may be accomplished by a number of ways. For example, the determination may be made by an enzyme-linked immunosorbent assay (ELISA), sodium dodecyl sulfate polyacrylamide gel electrophoresis (SDS-PAGE), Western blot analysis, and mass spectrometric analysis.

In other embodiments of the invention, an immune complex can be detected with a labeled secondary antibody, for example, that has specificity for a class determining portion of an anti-CBir1 antibody. A signal from a detectable secondary antibody can be analyzed and positive results indicate the presence of anti-CBir1 antibodies. Also disclosed are methods of treating Crohn's disease by the use of antigen-directed therapy. The target antigen in this therapy may be flagellin, and particularly CBir1 or an immunoreactive fragment thereof.

In other embodiments, methods are provided to define a subset of CD patients that may have colitic disease, and/or colitic and small bowel disease. Defining this subset of CD patients may be performed by determining the presence of anti-CBir1 expression and determining the presence of perinuclear antineutrophil cytoplasmic antibodies (pANCA), where the presence of both is diagnostic of Crohn's disease with properties of colitic disease and/or colitic and small bowel disease. Determination of the presence of pANCA may also be accomplished using ELISA, SDS-PAGE, Western blot analysis, or mass spectrometric analysis. These examples are not intended to be limiting, as one skilled in the art will recognize other appropriate means for determining the presence of pANCA.

Also disclosed are methods of treating the subset of CD patients with colitic disease and/or colitic and small bowel disease. Treating colitic disease and/or colitic and small bowel disease may be performed by manipulating the bacterial flora in the colon and/or colon and small bowel. Manipulation of the bacterial flora may be performed by administering antibiotics and/or probiotics.

Samples useful in various embodiments of the present invention can be obtained from any biological fluid having antibodies or RNA sequences or fragments of RNA sequences; for example, whole blood, plasma, serum, saliva, or other bodily fluid or tissue. The sample used in connection with various embodiments of the present invention may be removed from the mammal; for example, from drawn blood, aspirated fluids, or biopsies. Alternatively, the sample may be *in situ*; for example a tool or device may be used to obtain a sample and perform a diagnosis while the tool or device is still in the mammal.

A CBir1 antigen, or immunoreactive fragment thereof, useful in the invention can be produced by any appropriate method for protein or peptide synthesis.

Other embodiments of the present invention use anti-idiotypic antibodies specific to the anti-CBir1 antibody or other antibody of interest.

Disclosed are also kits for diagnosing and/or treating Crohn's disease and/or subtypes of Crohn's disease. The exact nature of the components configured in the kits depend on their intended purpose. For instance, a quantity of CBir1 antigen may be included in the kit for determining the presence of anti-CBir1 antibodies. Instructions for use may be included in the kit.

Other features and advantages of the invention will become apparent from the following detailed description, taken in conjunction with the accompanying drawings, which illustrate, by way of example, various features of embodiments of the invention.

### BRIEF DESCRIPTION OF THE FIGURES

Exemplary embodiments are illustrated in referenced figures. It is intended that the embodiments and figures disclosed herein are to be considered illustrative rather than restrictive.

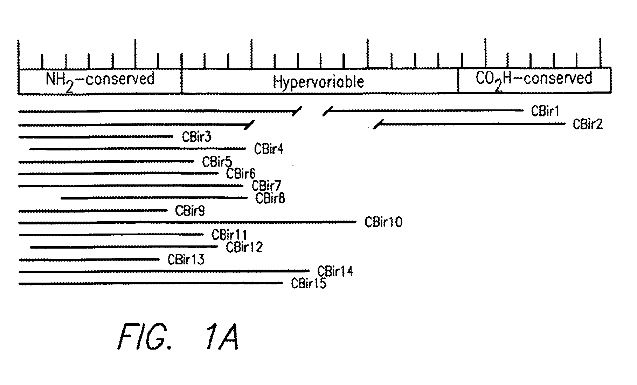

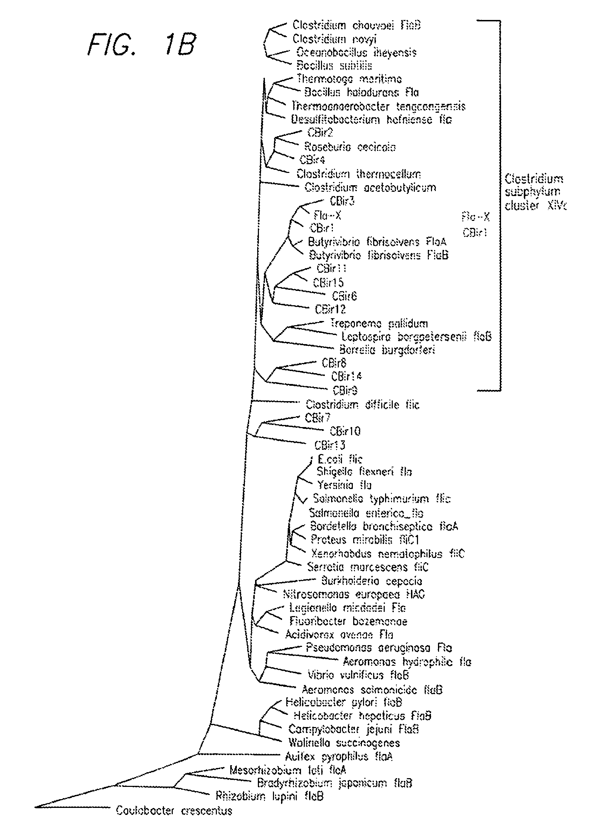

Figure 1 depicts the Flagellin clone identity and similarity to known flagellin sequences, in accordance with an embodiment of the present invention. (**A**) Schematic of CBir flagellin clones from serological expression screening. The predicted amino acid sequences from the flagellin expression clones (CBir1-CBir15) are mapped in relation to the representation of the *B. fibrisolvens* sequence at the top. Ruler length equals 500 amino acids. Similarity in the NH₂ conserved sequence between these flagellin clones and *B. fibrisolvens* sequences ranged from 45 to 84% (mean, 60.3%). Breaks in the lines representing clones CBir1 and CBir2 indicate differences in sequence length in the hypervariable region. NH₂-conserved, conserved NH₂ sequence; CO₂H-conserved, conserved carboxy sequence. (**B**) Phylogenetic tree showing relatedness at the conserved NH₂ termini of CBir1-CBir15 clones to flagellin sequences in the GenBank database. The dendrogram was constructed using the Clustal program in DNAStar and reflects similarity at the amino acid level. The approximate location of the *Clostridium* subphylum cluster XIVa is indicated with a bracket.

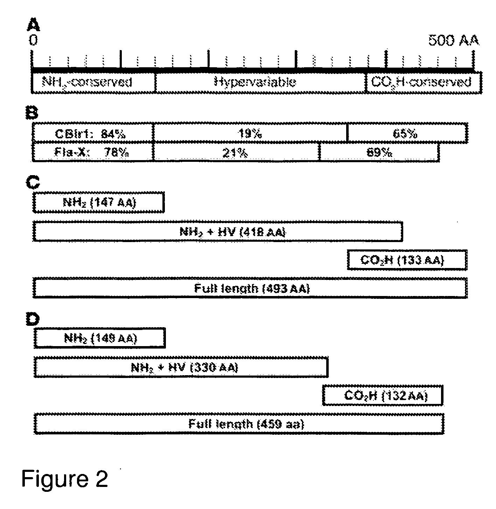

Figure 2 depicts the schematic of recombinant flagellin constructs with percent similarity to related flagellin B from the anaerobe *B. fibrisolvens* (GenBank accession number AAB82613), in accordance with an embodiment of the present invention. (**A**) Structure of *B. fibrisolvens* flagellin B showing conserved NH₂ and carboxy (CO₂H-conserved) regions and the hypervariable central domain. (**B**) Diagram of the full-length amino acid sequence of mouse cecal bacteria flagellins CBir1 and Fla-X, indicating the similarity of the three domains with the respective *B. fibrisolvens* domains. (**C** and **D**) Schematics of recombinant flagellin proteins and fragments for CBir1 (**C**) and Fla-X (**D**) expressed in *E. coli* and purified by six-histidine tag affinity to nickel-nitrilotriacetic acid columns.

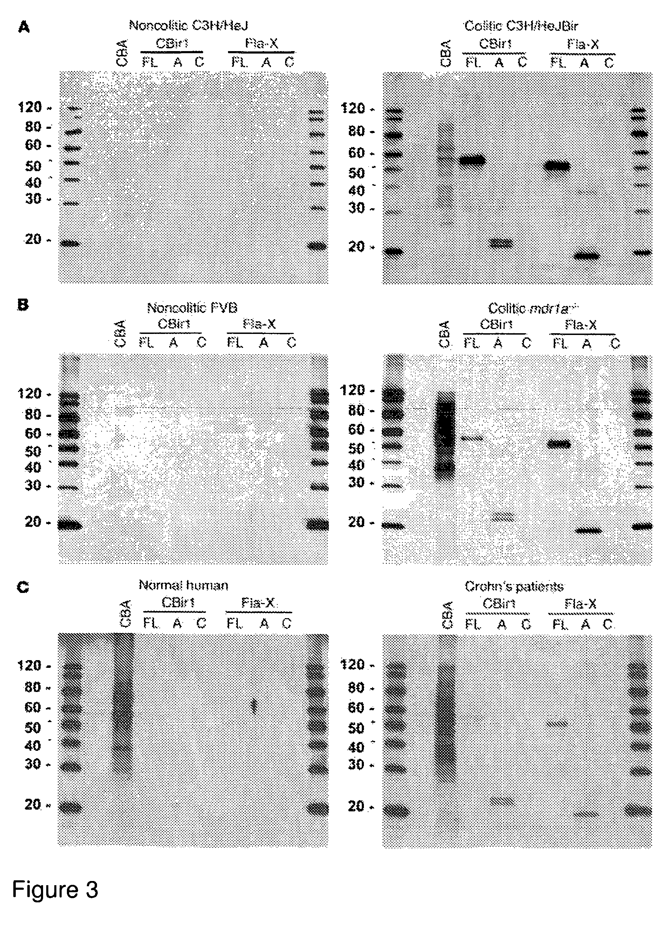

Figure 3 depicts the Western blot analysis of the serum antibody response to recombinant flagellins CBir1 and Fla-X and their fragments, in accordance with an embodiment of the present invention. (**A**) Noncolitic C3H/HeJ (pool of two) versus colitic C3H/HeJBir (pool of five) mice. (**B**) Noncolitic FVB (pool of five) versus colitic *mdr1a^{-l-}* (pool of five) mice. (**C**) Random human blood donor (Normal human) versus a pool of CD patients with severe disease. Protein samples include mouse CBA, full-length recombinant proteins (FL), the NH₂ conserved region (A) and the conserved carboxy region (C) of flagellin (see Figure 2, C and D).

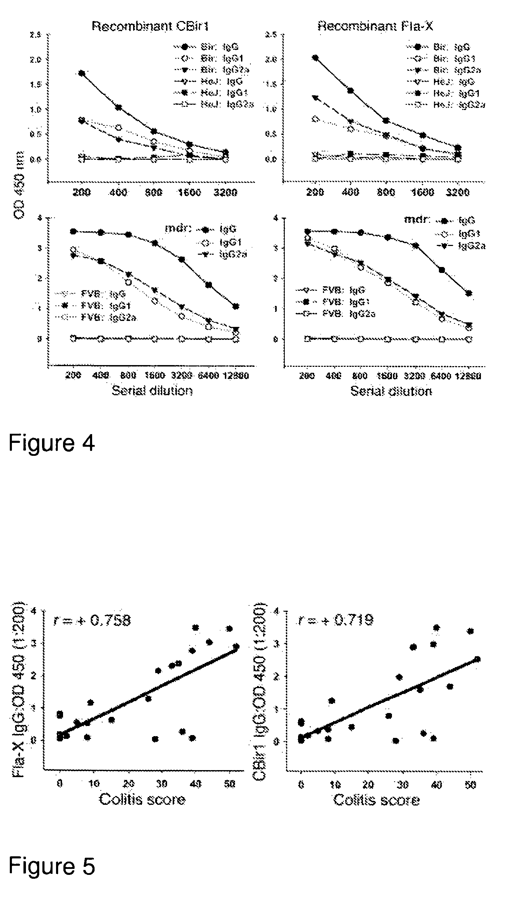

Figure 4 depicts the ELISA titration of mouse serum anti-flagellin against recombinant flagellins CBir1 and Fla-X with secondary antibodies specific for mouse IgG, IgG1, and IgG2a antibodies, in accordance with an embodiment of the present invention. Colitic C3H/HeJBir serum (pool of five) versus noncolitic C3H/HeJ serum (pool of two) was used in the upper panel and colitic *mdr1a*^{*-*/*-*} serum (pool of five) versus noncolitic FVB serum (pool of five) was used in the lower panel.
Figure 5 depicts the correlation of colitis histopathology score (0-60) with serum anti-Fla-X and anti-CBir1, in accordance with an embodiment of the present invention. Twenty-three *mdr1a*^{*-*/*-*} mice, ranging in age from 6 to 13 weeks, were randomly chosen for assignment of quantitative histopathology scores. Serum anti-flagellin from these mice was quantified by ELISA. Colitis scores of 0-2 represent no disease; 3-15, mild disease, 16-35, moderate disease, and more than 35, severe disease (Winstanley C, Morgan JAW. The bacterial flagellin gene as a biomarker for detection, population genetics and epidemiological analysis. Microbiology. 1997;143:3071-3084.). Similar results were obtained for both recombinant flagellins: Fla-X (left panel) and CBir1 (right panel).

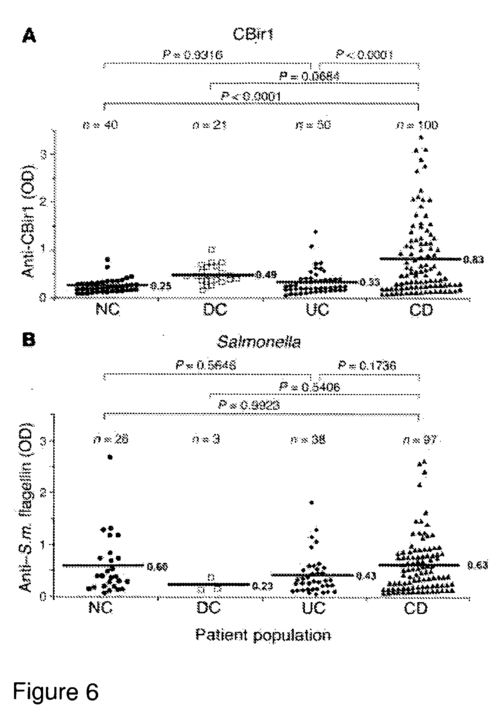

Figure 6 depicts the association of anti-flagellin antibodies with human IBDs, in accordance with an embodiment of the present invention. Human sera, well characterized for CD and UC, were tested by ELISA for reactivity to flagellin CBir1 (**A**) and *Salmonella muenchen (S.m.)* flagellin (**B**). Statistical analysis was performed with the Tukey-Kramer test; the resulting statistics (*P* values) as well as population size (n) are shown above the graphs. Mean OD₄₅₀ values are indicated by horizontal bars.

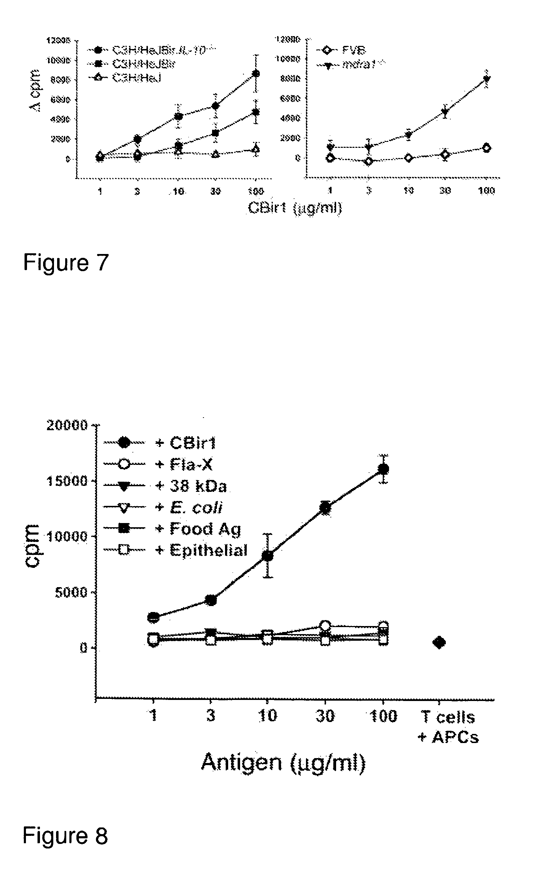

Figure 7 depicts the dose response of CD4⁺ T cell proliferation to CBir1 and Fla-X in multiple strains of mice, in accordance with an embodiment of the present invention. Left panel: C3H/HeJ (open triangles), C3H/HeJBir (squares), and C3H/HeJBir.IL-10^{-/-} (circles). Right panel: FVB (diamonds) and *mdr1a*^{*-*/*-*} (filled triangles). The *y* axes indicate sample counts per minute (cpm) minus control T cell plus APC cpm (Δ cpm) for each experimental group. The x axes indicate the dose (µg/ml) of recombinant flagellin used in each assay. Vertical bars indicate plus or minus one standard deviation of the mean value.
Figure 8 depicts the dose response and specificity of C3H/HeJBir CD4⁺ CBir1-specific T cell line, in accordance with an embodiment of the present invention. T cell line CBir-1 B1 proliferated specifically in response to recombinant flagellin protein CBir1. Antigens used in the assay include recombinant flagellins CBir1 (filled circles) and Fla-X (open circles); the 38-kDa antigen of *M*. *tuberculosis* (p38 antigen; 38 kDa: filled triangles); lysate of *E. coli* antigens (*E*. *coli*; open triangles); protein antigens extracted from mouse food pellets (Food Ag; filled squares); and a lysate of the ModeK epithelial cell line, of C3H origin (epithelial: open squares). Several randomly expressed recombinant commensal bacterial antigens were also tested and were negative (including randomly cloned C3H/HeJ mouse cecal bacterial antigens 99 [rIB99] and 32 [rIB32]). T cells plus APCs only are indicated by a filled diamond.

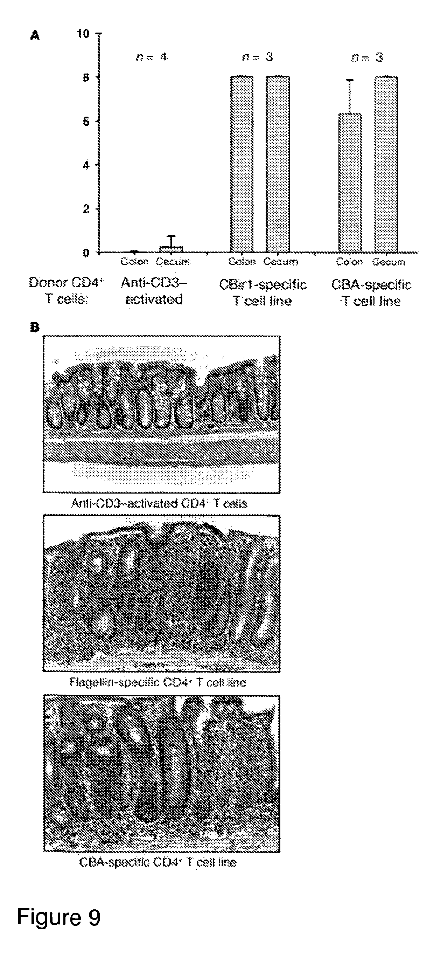

Figure 9 depicts the adoptive transfer of C3H/HeJBir CD4⁺ CBir1-specific T cell line into C3H/HeJ *scid*/*scid* recipients, in accordance with an embodiment of the present invention. (**A**) Two months after transfer, cecal and colon histopathology was assigned scores with a quantitative system (Cong Y, et al. CD4+ T cells reactive to enteric bacterial antigens in spontaneously colitic C3H/HeJBir mice: increased T helper cell Type 1 response and ability to transfer disease. J. Exp. Med. 1998;187:855-864.). CD4⁺ T cells activated polyclonally with mAb against CD3 prior to transfer were used as a negative control (Anti-CD3-activated). A CBA-specific CD4⁺ T cell line reactive with unselected cecal bacterial antigens was used as a positive control (CBA-specific T cell line); the CBir1-specific CD4⁺ T cell line corresponds to the flagellin-specific T cell line in Figure 8. Sample size (*n*) is indicated at the top. (**B**) Representative histopathology of the groups shown in **A**: Anti-CD3-activated CD4⁺ T cells (top panel), CBir1 flagellin-specific CD4⁺ T cells (middle panel), and CBA-specific CD4⁺ T cells (bottom panel). Magnification, x200.

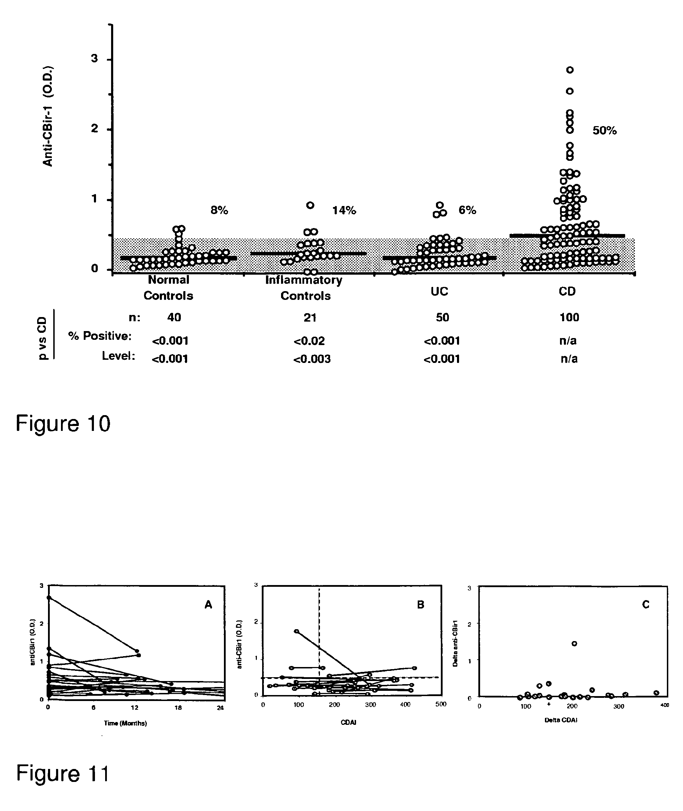

Figure 10 shows fifty percent of patients with Crohn's disease have antibodies to CBir1 and depicts the level of antibody response in Cohort 1 to CBir1 flagellin, in accordance with an embodiment of the present invention. The gray area indicates the negative range as defined by <2 SD above the mean of the normal controls, lines indicate the median level for each group. The percentage of positive samples for each group is shown. Wilcoxon signed rank test was used for assessing significance of number of positive samples, chi square analysis was used for significance of OD levels of positivity.
Figure 11 shows the change in antibody levels following surgery or infliximab therapy and depicts the relation of CBir1 antibody expression level to disease activity over time, in accordance with an embodiment of the present invention. A. Serologic responses towards CBir1 in 24 surgical CD patients at time of small bowel surgery (time 0) and at least 6 months or more after surgery. Dashed lines represent the demarcation between positive and negative values. B. CDAI and antibody expression levels for infliximab treated patients at two connected time points. C. Change in CDAI score and antibody expression level between the time points shown in B. The median change in CDAI and antibody expression level is depicted by a cross, ○ (open circle) = change in antibody expression from negative to positive or vice verso; ● (filled circle) = no change.

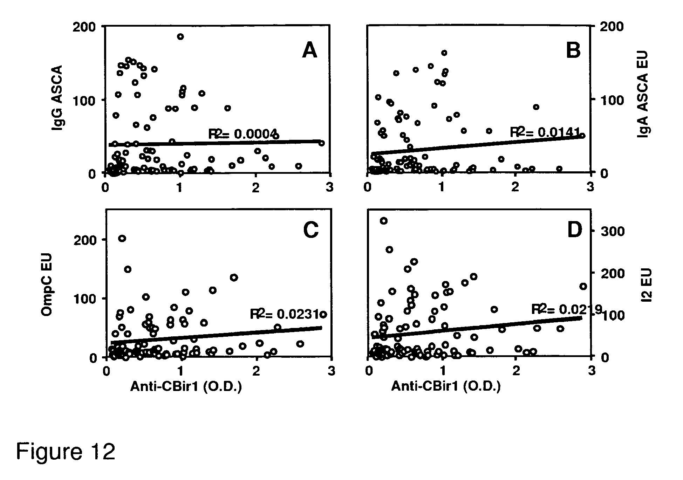

Figure 12 shows that the level of anti-CBir1 is independent of other serum markers and depicts the relationship between marker antibodies in CD by level of response, in accordance with an embodiment of the present invention. Correlation coefficients for linear fits are shown, p for all R²<0.05.

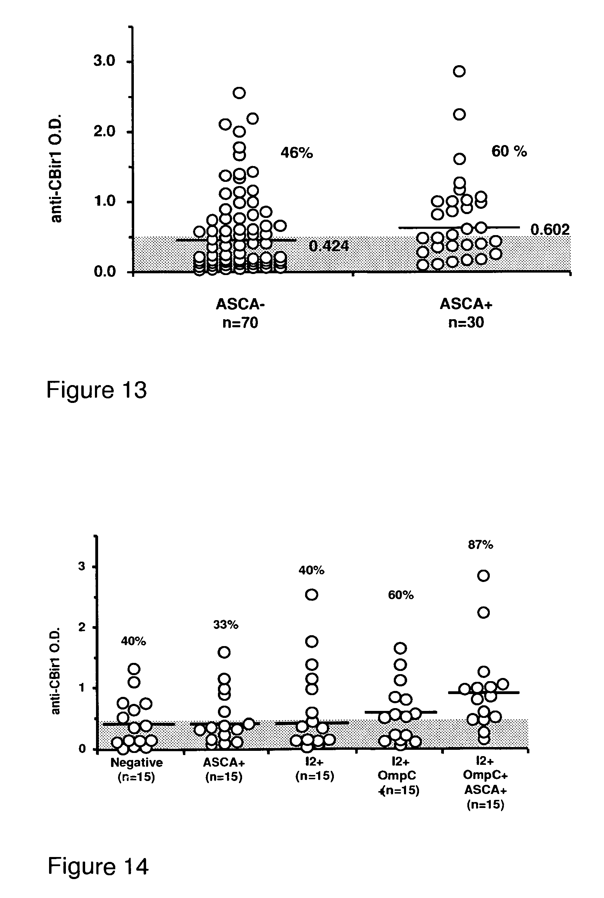

Figure 13 shows that anti-CBir1 is expressed in approximately 50% of ASCA-negative patients with Crohn's disease and depicts the level of antibody response in ASCA+ and ASCA- subsets of CD to CBir1 flagellin, in accordance with an embodiment of the present invention. The gray area indicates the negative range as defined by 2SD above the mean of the normal controls, lines indicate the median level for each group. The percentage of positive samples for each group is shown.
Figure 14 shows that anti-CBir1 is found in all Crohn's disease serologic subtypes, but is most prevalent in I2+/OmpC+/ASCA+ patients and depicts the level of antibody response in defined subsets of CD to CBir1 flagellin, in accordance with an embodiment of the present invention. Subsets are negative for all antibodies other than those listed. The gray area indicates the negative range as defined by 2SD above the mean of the normal controls, lines indicate the median level for each group. The percentage of positive samples for each group is shown.

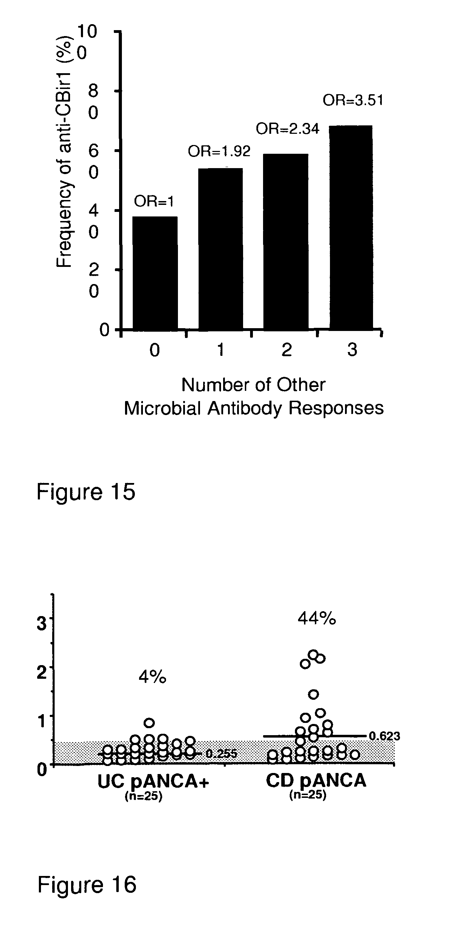

Figure 15 shows that the frequency of anti-CBir1 expression increases with multiple microbial antibody expression and depicts the frequency of anti-CBir expression in patients with no other microbial antibodies and those expressing 1, 2, or 3 other microbial antibodies (p trend <0.0005), in accordance with an embodiment of the present invention.
Figure 16 shows that forty-four percent of pANCA-positive patients with Crohn's disease are also positive for anti-CBir1 and depicts the level of antibody response to CBir1 flagellin in pANCA⁺ UC vs pANCA⁺ CD subsets, in accordance with an embodiment of the present invention. The gray area indicates the negative range as defined by 2SD above the mean of the normal controls, lines indicate the median level for each group. The percentage of positive samples for each group is shown.

### DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994); March, Advanced Organic Chemistry Reactions, Mechanisms and Structure 4th ed., J. Wiley & Sons (New York, NY 1992); Sambrook and Russel, Molecular Cloning: A Laboratory Manual 3rd ed., Cold Spring Harbor Laboratory Press (Cold Spring Harbor, NY 2001); and D. Lane, Antibodies: A Laboratory Manual (Cold Spring Harbor Press, Cold Spring Harbor NY, 1988), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

"Fibrostenosis" as used herein refers to a classification of Crohn's disease characterized by one or more accepted characteristics of fibrostenosing disease. Such characteristics of fibrostenosing disease include, for example, documented persistent intestinal obstruction or intestinal resection for an intestinal obstruction. The fibrostenosis can be accompanied by other symptoms such as perforations, abscesses or fistulae, and can be further characterized by persistent symptoms of intestinal blockage such as nausea, vomiting, abdominal distention and inability to eat solid food.

"Immune complex" and "complex" as used herein refer to an aggregate of two or more molecules that result from specific binding between an antigen and an antibody.

"Secondary antibody" means an antibody or combination of antibodies, which binds to the antibody of interest (i.e., the primary antibody); for example an antibody that binds to a pANCA or binds an antibody that specifically binds a CBir1 flagellin antigen, or an immunoreactive fragment thereof.

"Labeled secondary antibody" means a secondary antibody, as defined above, that can be detected or measured by analytical methods. Thus, the term labeled secondary antibody includes an antibody labeled directly or indirectly with a detectable marker that can be detected or measured and used in an assay such as an enzyme-linked immunosorbent assay (ELISA), fluorescent assay, radioimmunoassay, radial immunodiffusion assay or Western blotting assay. A secondary antibody can be labeled, for example, with an enzyme, radioisotope, fluorochrome or chemiluminescent marker. In addition, a secondary antibody can be rendered detectable using a biotin-avidin linkage such that a detectable marker is associated with the secondary antibody. Labeling of the secondary antibody, however, should not impair binding of the secondary antibody to the CBir1 antigen.

"Mammal" as used herein refers to any member of the class Mammalia, including, without limitation, humans and nonhuman primates such as chimpanzees, and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus adult and newborn subjects, as well as fetuses, whether male or female, are intended to be including within the scope of this term.

"Treatment" and "treating," as used herein refer to both therapeutic treatment and prophylactic or preventative measures, wherein the object is to prevent, slow down and/or lessen the disease even if the treatment is ultimately unsuccessful.

Various embodiments of the present invention provide for methods for diagnosing Crohn's disease in a mammal. Additional embodiments provide for determining a subtype of Crohn's disease, such as a phenotypic feature associated with Crohn's disease. Further embodiments provide for treating Crohn's disease. In a one embodiment, the mammal is a human.

In particular embodiments, diagnosing Crohn's disease may be performed by determining the presence of anti-CBir1 expression, where the presence of anti-CBir1 expression indicates that the mammal has Crohn's disease. Determining a subtype of Crohn's disease, such as a phenotypic feature associated with Crohn's disease may also be performed by determining the presence of anti-CBir1 expression, where the presence of anti-CBir1 indicates that the mammal has small bowel disease, internal penetrating/perforating disease or fibrostenosing disease.

Determining the presence of anti-CBir1 expression may be accomplished by various means. For example, determining the presence of anti-CBir1 expression may be performed by determining the presence of an RNA sequence or a fragment of an RNA sequence that encodes an anti-CBir1 antibody; for example, using Northern blot analysis or reverse transcription-polymerase chain reaction (RT-PCR). Determining the presence of anti-CBir1 expression may also be performed by determining the presence of anti-CBir1 antibodies; for example IgG anti-CBir1. Anti-CBir1 antibodies are not limited to IgG, as IgA, IgM, IgD and IgE are also included in various embodiments of the present invention. These examples are not intended to be limiting, as one skilled in the art will recognize other appropriate techniques for determining the presence of anti-CBir1 expression.

Determining the presence of anti-CBir1 antibodies may be accomplished by a number of ways. For example, the determination may be made by an enzyme-linked immunosorbent assay (ELISA), sodium dodecyl sulfate polyacrylamide gel electrophoresis (SDS-PAGE), Western blot analysis, and mass spectrometric analysis.

In other embodiments for the determination of the presence of anti-CBir1 antibodies, an immune complex can be detected with a labeled secondary antibody, for example, that has specificity for a class determining portion of an anti-CBir1 antibody. One skilled in the art understands that, preferably, a secondary antibody does not compete with the CBir1 flagellin antigen for binding to the primary antibody. A secondary antibody can bind any epitope of an anti-CBir1 antibody.

It is understood that a useful secondary antibody is specific for the species from which the sample was obtained. For example, if human serum is the sample to be assayed, mouse anti-human IgG can be a useful secondary antibody. A combination of different antibodies, which can be useful in the methods of the invention, also is encompassed within the meaning of the term secondary antibody, provided that at least one antibody of the combination reacts with an antibody that specifically binds a CBir1 antigen.

A secondary antibody can be rendered detectable by labeling with an enzyme such as horseradish peroxidase (HRP), alkaline phosphatase (AP), beta-galactosidase or urease. A secondary antibody also can be rendered detectable by labeling with a fluorochrome (such a fluorochrome emits light of ultraviolet or visible wavelength after excitation by light or another energy source), a chemiluminescent marker or a radioisotope.

A signal from a detectable secondary antibody can be analyzed, for example, using a spectrophotometer to detect color from a chromogenic substrate; a fluorometer to detect fluorescence in the presence of light of a certain wavelength; or a radiation counter to detect radiation, such as a gamma counter for detection of iodine-125. For detection of an enzyme-linked secondary antibody, for example, a quantitative analysis can be made using a spectrophotometer. If desired, the assays of the invention can be automated or performed robotically, and the signal from multiple samples can be detected simultaneously.

These examples are not intended to be limiting, as one skilled in the art will recognize other appropriate techniques for determining the presence of anti-CBir1 antibodies.

Also disclosed herein are ethods of treating Crohn's disease in a human by the use of antigen-directed therapy. The target antigen in this antigen-therapy may be flagellin, and particularly CBir1 or an immunoreactive fragment thereof.

In other embodiments, methods are provided to diagnose a subset of CD patients that may have colitic disease, and/or colitic and small bowel disease. Defining this subset of CD patients may be performed by determining the presence of anti-CBir1 expression and determining the presence of perinuclear antineutrophil cytoplasmic antibodies (pANCA), where the presence of both is diagnostic of Crohn's disease with properties of colitic disease and/or colitic and small bowel disease. Determination of the presence of pANCA may also be accomplished using ELISA, SDS-PAGE, Western blot analysis, or mass spectrometric analysis. These examples are not intended to be limiting, as one skilled in the art will recognize other appropriate means for determining the presence of pANCA.

Also disclosed are methods of treating the subset of CD patients with colitic disease and/or colitic and small bowel disease. Treating colitic disease and/or colitic and small bowel disease may be performed by manipulating the bacterial flora in the colon and/or colon and small bowel. Manipulation of the bacterial flora may be performed by administering antibiotics and/or probiotics. Examples of probiotics include but are not limited to *Bifidobacterium,* including, *B. bifidum, B. breve, B. infantis,* and *B. longum; Lactobacillus,* including, *L. acidophilus, L. bulgaricus, L. casei, L. plantarum, L. rhamnosus, L. reuiteri,* and *L. paracasei.*

Samples useful in various embodiments of the present invention can be obtained from any biological fluid having antibodies or RNA sequences or fragments of RNA sequences; for example, whole blood, plasma, serum, saliva, or other bodily fluid or tissue. It is understood that a sample to be assayed according to the various embodiments of the present invention may be a fresh or preserved sample obtained from a subject to be diagnosed. Furthermore, the sample used in connection with various embodiments of the present invention may be removed from the mammal; for example, from drawn blood, aspirated fluids, or biopsies. Alternatively, the sample may be *in situ;* for example a tool or device may be used to obtain a sample and perform a diagnosis while the tool or device is still in the mammal.

A CBir1 antigen, or immunoreactive fragment thereof, useful in the invention can be produced by any appropriate method for protein or peptide synthesis.

Other embodiments of the present invention use anti-idiotypic antibodies specific to the anti-CBir1 antibody or other antibody of interest. An anti-idiotypic antibody contains an internal image of the antigen used to create the antibody of interest. Therefore, an anti-idiotypic antibody can bind to an anti-CBir1 antibody or other marker antibody of interest. One skilled in the art will know and appreciate appropriate methods of making, selecting and using anti-idiotype antibodies.

Also disclosed are kits for diagnosing and/or treating Crohn's disease and/or subtypes of Crohn's disease. The kit is useful for practicing the inventive methods of diagnosing and/or treating Crohn's disease and/or subtypes of Crohn's disease. The kit is an assemblage of materials or components.

The exact nature of the components configured in the kit depends on its intended purpose. For example, some embodiments are configured for the purpose of diagnosing Crohn's disease or subtypes of Crohn's disease. Subtypes include small bowel disease, internal perforating disease, fibrostenosing disease, colitic disease and colitic and small bowel disease. For instance, a quantity of CBir1 antigen may be included in the kit for determining the presence of anti-CBir1 antibodies in accordance with various embodiments of the present invention. Additional embodiments are configured for treating Crohn's disease or subtypes of Crohn's disease. Further embodiments are configured for treating Crohn's disease patients with colitic disease and/or colitic and small bowel disease. In one embodiment, the kit is configured particularly for the purpose of diagnosing human subjects. In another embodiment, the kit is configured particularly for the purpose of treating human subjects.

Instructions for use may be included in the kit. "Instructions for use" typically include a tangible expression describing the technique to be employed in using the components of the kit to effect a desired outcome, such as to diagnose or treat Crohn's disease and/or subtypes of Crohn's disease. Optionally, the kit also contains other useful components, such as, secondary antibodies, enzymes (e.g., horseradish peroxidase (HRP), alkaline phosphatase (AP), beta-galactosidase or urease), fluorochrome, chemiluminescent markers, radioisotopes, labeled secondary antibodies, tetramethylbenzidine substrates, multiple well plates, diluents, buffers, pharmaceutically acceptable carriers, syringes, catheters, applicators, pipetting or measuring tools, or other useful paraphernalia as will be readily recognized by those of skill in the art.

Chronic intestinal inflammation, as seen in inflammatory bowel disease (IBD), results from an aberrant and poorly understood mucosal immune response to the microbiota of the gastrointestinal tract in genetically susceptible individuals. Serological expression cloning to identify commensal bacterial proteins that could contribute to the pathogenesis of IBD was used. The dominant antigens identified were flagellins, molecules known to activate innate immunity via Toll-like receptor 5 (TLR5), and critical targets of the acquired immune system in host defense. Multiple strains of colitic mice had elevated serum anti-flagellin IgG2a responses and Th1 T cell responses to flagellin. In addition, flagellin-specific CD4⁺T cells induced severe colitis when adoptively transferred into naive SCID mice. Serum IgG to these flagellins, but not to the dissimilar *Salmonella muenchen* flagellin, was elevated in patients with Crohn's disease, but not in patients with ulcerative colitis or in controls. These results identify flagellins as a class of immunodominant antigens that stimulate pathogenic intestinal immune reactions in genetically diverse hosts and suggest new avenues for the diagnosis and antigen-directed therapy of patients with IBD.

A molecular technique known as serological expression cloning (SEC) was used to identify specific bacterial antigens driving experimental IBD. SEC involves the screening of DNA expression libraries in lambda phage with defined antisera.

Molecular cloning of antigens by SEC using sera from colitic C3H/HeJBir mice is described. The dominant antigens identified were a family of related, novel flagellins. Strong reactivity against specific flagellins was seen in multiple models of experimental colitis across several distinct MHC haplotypes. There was a clear IgG2a predominance to the anti-flagellin response, suggesting a concurrent Th1-biased T cell response against flagellin. Indeed, marked reactivity against flagellin was seen in mesenteric and splenic T cell cultures from colitic animals, and flagellin-specific T cells were able to induce colitis when adoptively transferred into immunodeficient animals. Surprisingly, the reactivity against these flagellins (but not against the dissimilar *Salmonella* flagellin) was also seen in human IBD sera, with significant reactivity in patients with CD but not UC or control patients.

Using an unbiased molecular screen to search for bacterial antigens relevant to IBD, the dominant antigens identified were a family of related, novel flagellins. A strong, IgG2a-biased serological response to these specific flagellins was seen in multiple models of experimental colitis across several distinct MHC haplotypes. In addition, marked reactivity against these flagellins was seen at the T cell level, and flagellin-specific T cells were able to induce colitis when adoptively transferred into immunodeficient animals. Interestingly, while these flagellins were identified from mouse cecal bacteria, there was clear, specific reactivity against these molecules in patients with CD (but not in patients with UC or in NCs).

It has been observed that full-length flagellin Fla-X (endotoxin free) is capable of stimulating TNF-α production by human macrophages in vitro (M.J. Lodes and R.M. Hershberg). It is tempting to speculate that the intrinsic "adjuvanticity" of flagellin is likely to contribute to its antigenicity. While flagellin molecules clearly have the capacity to stimulate the production of proinflammatory cytokines via TLR5, and while not wishing to be bound to any particular theory, it is believed that the B and T cell responses to flagellin contribute more directly to the chronic intestinal inflammation seen in IBD.

The clinical data (Figure 6) are consistent with the fact that the aberrant response in patients with CD is specific to the subgroup of flagellins identified in the inventive molecular screen. Specifically, there was no correlation between IBD and a response to flagellin from *Salmonella muenchen,* which is very similar (84-91%) in the NH₂ conserved region to the flagellin from the commensal organism *Escherichia coli.* It must be emphasized that the flagellins identified were from a source of material devoid of known bacterial pathogens. The bacteria with genes that "encode" the flagellins CBir1 and Fla-X (the two dominant flagellins tested) are unknown; however, preliminary phylogenetic data suggest that these flagellins are most closely related to the flagellins of bacteria in the genera *Butyrivibrio, Rosburia, Thermotoga,* and *Clostridium* and fall within the *Clostridium* subphylum XIVa cluster of Gram-positive bacteria (Figure 1B). While not wishing to be bound to any particular theory, it is believed that the aberrant response to the flagellin molecule(s) from these organisms is related to a combination of the intrinsic property of the molecules themselves (as immunogens and adjuvants) and an underlying genetic susceptibility. Using monoclonal antibodies directed against CBir1, the inventors have demonstrated that this antigen is present in the stool of wild-type strains (FVB, C57BL/6, BALB/c, and C3H/HeJ) and colitic strains (*mdr1a^{-l-},* B6.IL-10^{-/-}, and C3H/HeJBir). These data indicate that the presence of the antigen itself does not strictly correlate with colitis. Still, the widespread presence of these antigens does not preclude the possibility of enhanced colonization of organisms expressing these flagellins in CD lesions.

In general, the data are consistent with the belief that IBD is associated with a defect in tolerance to commensal organisms (Duchmann R, et al. Tolerance exists towards resident intestinal flora but is broken in active inflammatory bowel disease. Clin. Exp. Immunol. 1995;102:448-455.). The IgG2a-biased antibody against Fla-X and CBir1 highlights the Th1 bias of the T cell responses seen. The broad recognition of these flagellins in several different mouse models and in humans with CD indicates that these flagellins are among the immunodominant antigens of the microbiota. However, the exact role of these flagellins in the pathogenesis of IBD (e.g., whether they are predominant or obligatory for disease) compared with that of other microbial antigens remains to be defined. While not wishing to be bound to any particular theory, it is believed that a T cell regulatory response to specific flagellins (and/or other bacterial antigens) may be selectively impaired in IBD. In this context, specific flagellin molecules may represent novel targets for antigen-directed therapy in IBD.

As observed with the specific flagellins identified here, only a subset of patients with CD show specific seroreactivity against I2 (an antigen derived from *Pseudomonas fluorescens*).

Antibody responses to certain microbial antigens define heterogeneous groups of Crohn's patients; multiple and high-level responses to these antigens are associated with aggressive clinical phenotypes. The flagellin, CBir1, identified by the inventors in the C3H/HeJBir mouse model, is a dominant antigen capable of inducing colitis in mice and eliciting antibody responses in a subpopulation of patients with Crohn's disease. Serum response to CBir1 flagellin in Crohn's disease patients was evaluated and compared to previously defined responses to oligomannan (ASCA), I2, OmpC and neutrophil nuclear autoantigens (pANCA), and to determine anti-CBir1 associated phenotypes.

It was found that the presence and level of IgG anti-CBir1 were associated with Crohn's disease, independently. Anti-CBir1 was present in all antibody subgroups and expression increases in parallel with increases in the number of antibody responses. pANCA⁺ Crohn's patients were more reactive to CBir1 than were pANCA⁺ ulcerative colitis patients. Anti-CBir1 expression is independently associated with small bowel, internal-penetrating and fibrostenosing disease features.

Thus, serum responses to CBir1 independently identify a unique subset of patients with complicated Crohn's disease. This is the first bacterial antigen identified in a murine model with a similar pattern of aberrant reactivity in a subset of Crohn's disease patients.

Serologic expression cloning was used to identify an immunodominant antigen, CBir1 flagellin, to which strong B cell and CD4⁺ T cell responses occur in colitic mice. Transfer of CBir1 specific CD4⁺ Th1 T cells to C3H/SCID mice generated a severe colitis dependent on endogenous expression of CBir1 flagellin in the cecum and colon. These findings prove that CBir1 flagellin is an immunodominant antigen of the enteric microbial flora. Of note, approximately 50% of patients with CD had serum reactivity to CBir1, whereas patients with ulcerative colitis, patients with other inflammatory GI diseases, and control subjects had little or no reactivity to this flagellin. The inventors determined the relationship of serum reactivity to CBir1 and the previously defined responses to oligomannan (ASCA), OmpC, I2 and pANCA in patients with CD and to define distinct clinical phenotypes. Results show that antibodies to CBir1 are independently associated with CD, have no correlation to levels of previously defined antibodies, are expressed in ASCA-negative and pANCA⁺ CD patients and are independently associated with aspects of complicated CD.

Investigations have yielded compelling evidence that serum antibody to CBir1 flagellin, marks for an independent subset of patients with CD. It is shown that the level of response can vary widely, that these responses are relatively stable over time and do not correspond with active or remission disease states. It is believed that anti-CBir1 expression is independent of serologic responses to previously defined bacterial antigens and is independently associated with complicated CD. It is also the first antigen to be discovered with a role as ligand for activation of the innate immune response via Toll-like receptors and a strong immunogen for adaptive immunity. This dual effect provides a focus for investigations of the role of anti-CBir1 in the pathogenesis of this subset of patients with CD.

It has been previously shown that groups of patients with unique disease characteristics can be distinguished by the presence and level of serum antibodies to one, two or all of the following antigens: oligomannan (ASCA); the novel Crohn's related bacterial sequence, I2; and E. coli outer-membrane porin-C (OmpC). While each of these reactivities may serve to subclassify phenotypes within CD, none of them have yet been shown to have any direct pathophysiologic significance. The dominant serologic immune response to CBir1 flagellin was found by serologic expression cloning using sera from colitic mice to screen a DNA phage library derived from mouse cecal bacteria. CBir1 flagellin was then used to generate a specific CD4⁺Th1 cell line. Transfer of this Th1 cell line into SCID mice induced a colitis due to reactivity to endogenous CBir1 flagellin in the microbial flora indicating that CBir1 is an immunodominant antigen in mouse colitis. CBir1 is the first bacterial antigen capable of inducing colitis in animals that demonstrates a similar aberrant immune response in patients with CD.

Interesting findings resulted from the examination of the relationship of anti-CBir1 to the previously defined antibodies to microbial antigens in patients with CD. The level of response to CBir1 is greater in patients who have increasing levels of reactivity to ASCA, OmpC, and I2 (with a peak occurring in those who respond to all three), which is consistent with the concept that this subset of patients has a propensity to respond to multiple bacterial antigens. However, high CBir1 reactivity was seen across all antibody-defined subsets, which is consistent with it being independent of the other antibody responses.

The data presented herein show that the serotypic and phenotypic associations with anti-CBir1 expression, (small bowel, internal penetrating, and fibrostenosing disease) differ from those associated with any or a combination of responses to I2, OmpC, oligomannan, or neutrophil nuclear antibodies. The lack of relationship to small bowel surgery and to ulcerative colitis-like suggest that to define the true phenotype associated with this antibody response may require further more precise clinical groupings.

Another seroreactivity that defines a subgroup of patients is pANCA, which is predominantly associated with ulcerative colitis and may reflect cross reactivity to bacteria (Seibold F, Brandwein S, Simpson S, Terhorst C, Elson CO. pANCA represents a cross-reactivity to enteric bacterial antigens. J Clin Immunol 1998;18:153-60.); however, there is a subset of patients with CD who also express pANCA (Vasiliauskas EA, Plevy SE, Landers CJ, Binder SW, Ferguson DM, Yang H, Rotter JI, Vidrich A, Targan SR. Perinuclear antineutrophil cytoplasmic antibodies in patients with Crohn's disease define a clinical subgroup. Gastroenterology 1996;110:1810-9. Vasiliauskas EA, Kam LY, Karp LC, Gaiennie J, Yang H, Targan SR. Marker antibody expression stratifies Crohn's disease into immunologically homogeneous subgroups with distinct clinical characteristics. Gut 2000;47:487-96.). pANCA⁺ CD patients have both colitic and left-sided disease with features similar to ulcerative colitis. Among the population of CD patients who express pANCA but do not react to the other known antigens, 40-44% expressed anti-CBir1, while anti-CBir1 expression was found in only 4% of pANCA⁺ ulcerative colitis patients. Because anti-CBir1 expression appears to be associated with a specific CD subtype, it may prove to be useful in distinguishing among patients with indeterminate colitis; i.e, those that may be more Crohn's-like compared to those that may be more ulcerative colitis-like. Used in combination with pANCA, anti-CBir1 expression may also be used diagnose a subset of patients with colitic and/or colitic *and* small bowel disease, perhaps defining those patients potentially likely to respond to manipulation of bacteria using either antibiotics or probiotics.

The expression of antibodies to CBir1 is indicative of an adaptive immune response to this antigen. Antibody reactivity to flagellin may provide an important tool to define potential differences in pathophysiologic immune mechanisms in innate and adaptive immunity in a subset of patients with CD. Anti-CBir expression defines a subgroup of CD patients not previously recognized by other serologic responses and is independently associated with aspects of the complicated CD phenotype. These results represent the first example of discovery from animal models having direct correlates in human disease.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1

### Isolation of genomic DNA of mouse cecal bacterium.

Pelleted bacteria from C3H/HeJBir mouse ceca were inactivated at 80°C for 20 minutes and then were treated with 2 ml lysozyme (20 mg/ml in Tris-EDTA [TE] buffer) for 1 hour at 37 °C. This solution was rocked at room temperature for 10 minutes with 40 µl proteinase K (10 mg/ml) and 140 µl 20% SDS (Sigma-Aldrich, St. Louis, Missouri, USA) and then incubated for 15 minutes at 65°C, then 0.4 ml of 5 M NaCl and 0.32 ml of a 10% cetyltrimethylammonium bromide (CTAB) solution (1 g CTAB [Sigma-Aldrich], 1.4 ml 5M NaCl, and 8.6 ml distilled H₂O) was added, followed by incubation at 65°C for 10 minutes. DNA was then extracted twice with phenol, followed by extraction with phenol/chloroform/isoamyl alcohol (24:24:2), and then with chloroform. Finally the DNA was precipitated with 0.6 volumes of isopropanol and resuspended in TE buffer.

### Example 2

### Genomic expression library construction

A detailed description of library construction can be found in the following references: Lodes, M.J., Dillon, D.C., Houghton, R.L., and Skeiky, Y.A.W. 2004. Expression cloning. In Molecular diagnosis of infectious diseases. 2nd edition. J. Walker, series editor; J. Decker and U. Reischl, volume editors. Humana Press. Totowa, New Jersey, USA. 91-106. Briefly, 20 µg of genomic DNA of mouse cecal bacterium was resuspended in 400 µl of TE buffer and was sonicated for five seconds at 30% continuous power with a Sonic Dismembrator (Fisher Scientific, Pittsburgh, Pennsylvania, USA) to generate fragments of approximately 0.5-5.0 kb. DNA fragments were blunted with T4 DNA polymerase (Invitrogen, Carlsbad, California, USA) and were ligated to *Eco*RI adaptors (Stratagene, La Jolla, California, USA) with T4 DNA ligase (Stratagene). Adapted inserts were then phosphorylated with T4 polynucleotide kinase (Stratagene) and were selected by size with a Sephacryl 400-HR column (Sigma-Aldrich). Approximately 0.25 µg of insert was ligated to 1.0 µg Lambda ZAP Express Vector treated with *Eco*RI and calf intestinal alkaline phosphatase (Stratagene), and the ligation mix was packaged with Gigapack III Gold packaging extract (Stratagene) following the manufacturer's instructions.

### Example 3

### Expression screening

Immunoreactive proteins were screened from approximately 6 x 10⁵ plaque-forming units (PFU) of the unamplified cecal bacterium expression lambda library. Briefly, twenty 150-mm petri dishes were plated with *E. coli* XL1-Blue MRF' host cells (Stratagene) and approximately 3 x 10⁴ PFU of the unamplified library and were incubated at 42 °C until plaques formed. Dry nitrocellulose filters (Schleicher and Schuell, Keene, New Hampshire, USA), pre-wet with 10 mM isopropyl β-thiogalactopyranoside (IPTG), were placed on the plates, which were then incubated overnight at 37°C. Filters were removed and washed three times with PBS containing 0.1% Tween 20 (PBST) (Sigma-Aldrich), blocked with 1.0% BSA (Sigma-Aldrich) in PBST, and washed three times with PBST. Filters were next incubated overnight with *E. coli* lysate-adsorbed C3H/HeJ Bir mouse serum (1:200 dilution in PBST), washed three times with PBST, and incubated with a goat anti-mouse IgG + IgA + IgM (heavy and light chain) alkaline phosphatase-conjugated secondary antibody (diluted 1:10,000 with PBST; Jackson Laboratories, West Grove, Pennsylvania, USA) for 1 hour. Filters were finally washed three times with PBST and two times with alkaline phosphatase buffer (pH 9.5) and were developed with nitroblue tetrazolium chloride/5-bromo-4-chloro-3-indolylphosphate *p*-toluidine salt (Invitrogen). Reactive plaques were then isolated and a second or third plaque purification was performed. Excision of phagemid followed the Stratagene Lambda ZAP Express protocol, and the resulting plasmid DNA was sequenced with an automated sequencer (ABI, Foster City, California, USA) using M13 forward, reverse, and sequence-specific internal DNA sequencing primers. Nucleic acid and predicted protein sequences were used to search the GenBank nucleotide and translated databases. Protein analysis was performed with the PSORT program (National Institute for Basic Biology, Okazaki, Japan) and with the IDENTIFY program of EMOTIF (Department of Biochemistry, Stanford University). Sequence alignments were produced with the MegAlign program (Clustal) of DNAStar (Madison, Wisconsin, USA). Note that 20 random clones from the lambda library were picked and sequenced prior to serolological expression cloning. None of the 20 were found to be derived from mouse DNA and no flagellins were identified.

### Example 4

### Cloning of full-length flagellins representing clones CBir1 and Fla-X

Clone CBir1 contains the conserved NH₂ and variable regions of an unknown immunoreactive flagellin. The full-length sequence was obtained by first amplifying the unknown CBir1 carboxy terminus from total genomic cecal bacterium DNA with Expand polymerase (Roche, Indianapolis, Indiana, USA) and the primers CBir1 var1 (designed from the variable region of CBir1; CACAATCACAACATCTACCCAG; SEQ. ID. NO.: 1) and CBir1 Carb Z (designed from the carboxy terminus of the related flagellin B of *Butyrivibrio fibrisolvens,* GenBank accession number AF026812; 5'-TTACTGTAAGAGCTGAAGTACACCCTG-3'; SEQ. ID. NO.: 2). This PCR product was cloned with a Zero Blunt TOPO PCR Cloning Kit (Invitrogen), digested with *Eco*RI, and gel-isolated (carboxy end of CBir1). Clone CBir1 plasmid DNA, which represents the NH₂ terminus plus flagellin central variable region and overlaps with the cloned carboxy region, was digested with *Sca*I and then gel-isolated. Both overlapping (181-bp) DNA fragments (approximately 20 ng each) were added to a PCR reaction with the primers CBir1 HIS and CBir1 TERMX (see below), and the amplification product was cloned and expressed as described below.

Fla-X is an immunoreactive full-length flagellin sequence with no known identity in the public databases. Full-length flagellin Fla-X was cloned from total cecal bacterium genomic DNA by PCR amplification with the primers CBir Fla-X HIS (5'-CAATTACATATGCATCACCATCACCATCACGTAGTACAGCACAATC-3'; SEQ. ID. NO.: 3) and CBir1 TERMX (5'-ATAGACTAAGCTTACTGTAAGAGCTGAAGTACACCCTG-3'; SEQ. ID. NO.: 4), and was expressed as described below. The amplification product was cloned with a Zero Blunt TOPO PCR Cloning Kit (Invitrogen), and several clones were sequenced.

### Example 5

### Recombinant protein.

Recombinant *Salmonella muenchen* flagellin (≥95% pure by SDS-PAGE) was obtained from Calbiochem (La Jolla, California, USA). Expression of other recombinant flagellin proteins and deletion constructs was accomplished by amplification from the cloned plasmid or genomic DNA (full length Fla-X) with *Pfu* polymerase (Stratagene) and the following primer pairs: for full-length CBir1, CBir1 HIS (5'-CAATTACATATG *CATCACCATCACCA TCA*CGTAGTACAGCACAATTTACAGGC-3'; SEQ. ID. NO.: 5) and CBir1 TERMX (5'-ATAGACTAAGCTTACTGTAAGAGCTGAAGTACACCCTG-3'; SEQ. ID. NO.: 6); for the CBir1 NH₂ plus variable regions, CBir1 HIS and CBir1 AV TERM (5'-ATAGACTAAGCTTAAGAAACCTTCTTGATAGCGCCAG-3'; SEQ. ID. NO.: 7); for the CBir1 NH₂ terminus, CBir1 HIS and CBir1 A TERM (5'-TAGACTGAATTCTAGTCCATAGCGTCAACGTTCTTTGTGTC-3'; SEQ. ID. NO.: 8); for the CBir1 carboxy terminus, CBir1 C HIS (5'-CAATTACATATG*CATCACCATCACCATCAC*AAGATGAACTTCCATGTAGGTGC-3'; SEQ. ID. NO.: 9) and CBir1 TERMX; for full-length Fla-X, CBir Fla-X HIS (5'-CAATTACATATG*CATCACCATCACCATCAC*GTAGTACAGCACAATC-3'; SEQ. ID. NO.: 10) and CBir1 TERMX (ATAGACTAAGCTTACTGTAAGAGCTGAAGTACACCCTG-3'; SEQ. ID. NO.: 11); for the Fla-X NH₂ plus variable regions, Fla-X HIS (5'-CAATTACATATG*CATCACCATCACCATCAC*GTAGTACAGCACAATCTTAGAGC-3'; SEQ. ID. NO.: 12) and Fla-X AV TERM (5'-ATAGACTAAGCTTAGAGGCTGAAATCAATGTCCTCG-3'; SEQ. ID. NO.: 13); for the Fla-X NH₂ terminus, Fla-X HIS and Fla-X A TERM (5'-ATAGACTAAGCTTAATGTGCTGAAAGATATCTTGTCAC-3'; SEQ. ID. NO.: 14); and for the Fla-X carboxy terminus, Fla-X C HIS (5'-CAATTACATATG*CATCACCATCACCATCAC*TTCAGCCTCCATGTAGGTGCAGATGC-3'; SEQ. ID. NO.: 15) and CBir1 TERMX. Primers contained restriction sites for cloning (in bold) and a six-histidine tag (in italics) for protein purification (NH₂ terminus). The amplification products were digested with the restriction enzymes *Nde*I and *Hind*III or *Eco*RI, depending on the primer set used, gel-isolated, and ligated to a pET 17b plasmid vector (Novagen, Madison, Wisconsin, USA) previously cut with *Nde*I and with *Hind*III or *Eco*RI and dephosphorylated with alkaline phosphatase (MB grade; Roche). The ligation mix was transformed into XL1 Blue competent cells (Stratagene) and plasmid DNA was prepared for sequencing (Qiagen, Valencia, California, USA). Recombinant protein was expressed by transformation of plasmid DNA into BL21 pLysS competent cells (Novagen) and induction of a single-colony cell culture with 2 mM IPTG (Sigma-Aldrich). Recombinant protein was recovered from cell lysate with nickel-nitrilotriacetic acid agarose beads (Qiagen), following the manufacturer's instructions, and was dialyzed in 10 mM Tris, pH 4-11 depending on predicted recombinant pl characteristics. Recombinant proteins were "quality-checked" for purity by SDS-PAGE followed by staining with Coomassie blue and by NH₂-terminal protein sequencing, and were quantified with a Micro BCA assay (Pierce, Rockford, Illinois, USA). Recombinants were assayed for endotoxin contamination with the Limulus assay (Bio Whittaker, Walkersville, Maryland, USA). Production of the *Mycobacterium tuberculosis* 38-kDa protein has been described previously (Lodes MJ, et al. Serodiagnosis of human granulocytic ehrlichiosis by using novel combinations of immunoreactive recombinant proteins. J. Clin. Microbiol. 2001;39:2466-2476.).

### Example 6

### ELISA

Ninety-six-well EIA/RIA microtiter plates (3369; Corning Costar, Cambridge, Massachusetts, USA) were coated overnight at 4°C with 100 ng/well of the recombinant proteins. Solutions were then aspirated from the plates, which were then blocked for 2 hours at room temperature with PBS containing 1% (weight/volume) BSA. This was followed by washing in PBST. Serum diluted in PBS containing 0.1% BSA was added to wells and incubated for 30 minutes at room temperature, followed by washing six times with PBST and then incubation with secondary antibody-HRP conjugate (1:10,000 dilution) for 30 minutes. Plates were then washed six times in PBST and then were incubated with tetramethylbenzidine (TMB) substrate (Kirkegaard and Perry, Gaithersburg, Maryland, USA) for 15 minutes. The reaction was stopped by the addition of 1 N sulfuric acid, and plates were "read" at 450 nm using an ELISA plate reader (Biotek instrument EL311, Hyland Park VA). Background values were determined by reading of reactions that lacked the primary antibody step.

### Example 7

### Western blot analysis

Recombinant antigens (50-200 ng/lane) were subjected to SDS-PAGE analysis using 15% polyacrylamide "minigels." The antigens were transferred to nitrocellulose BA-85 (Schleicher & Schuell, Keene, New Hampshire, USA) and were blocked for 1 hour at room temperature with PBS containing 1% Tween 20. Blots were then washed three times, 10 minutes each wash, in PBST. Next, blots were probed for 1 hour at room temperature with serum diluted 1:500 in PBST followed by washing three times, 10 minutes each wash, in PBST. Blots were then incubated for 30 minutes at room temperature with secondary antibody-HRP diluted 1:10,000 in wash buffer and were again washed three times for 10 minutes each wash in PBST containing 0.5 M sodium chloride. Finally, blots were incubated in chemiluminescent substrate for ECL (Amersham Plc, Little Charlton, UK) for about 1 minute and then were exposed to X-ray film (XAR5) for 10-60 seconds, as required.

### Example 8

CD4⁺ T cell isolation and culture, and generation of a Cbir1-specific T cell line.

CD4⁺ T cells were isolated from mesenteric lymph nodes (MLNs) of mice with BD IMAG anti-mouse CD4 beads according to the manufacturer's instructions (BD Biosciences Pharmingen, San Diego, California, USA). Briefly, MLN cells were labeled with anti-CD4 beads and then were placed within the magnetic field of the BD Imagnet. The unlabeled cells in suspension were removed and the cells binding to beads were washed and used in the CD4⁺ T cell culture. More than 99% of cells were CD4⁺, as shown by flow cytometry. For the generation of a T cell line reactive to CBir1, CD4⁺ T cells were isolated from MLNs of C3H/HeJBir mice as described above and were cultured with splenic APCs that were pulsed with CBir1 (100 mg/ml) overnight. The cells were restimulated every 10-14 days.

### Example 9

### Antigen-specific proliferation of T cells

Spleen and MLN CD4⁺ T cells, isolated as described above, or a CBir1 flagellin-specific T cell line (4 x 10⁵ cells/well) were incubated in triplicate in the presence of antigen-pulsed, irradiated APCs (4 x 10⁵ cells per well; treated with 1-100 µg/ml antigen for 18 hours at 37°C) in 96-well flat-bottomed tissue culture plates (Falcon, Lincoln Park, New Jersey, USA) at 37°C in 5% CO₂ humidified air. [³H]thymidine (0.5 µCi) (New England Nuclear, Boston, Massachusetts, USA) was added at day 3 of culture and the cells were harvested at 16 hours after the pulse. The cells were harvested on glass fiber filters on a PHD cell harvester (Cambridge Technology Inc., Watertown, Massachusetts, USA), washed with distilled water, and dried. Proliferation was assessed as the amount of incorporation of [³H]thymidine into cell DNA, as measured by beta scintillation counting (Beckman Instruments, Palo Alto, California, USA) of the harvested samples, and was expressed as cpm ± SD. The preparation of epithelial cell proteins and food antigens has been described previously (Cong Y, et al. CD4+ T cells reactive to enteric bacterial antigens in spontaneously colitic C3H/HeJBir mice: increased T helper cell Type 1 response and ability to transfer disease. J. Exp. Med. 1998;187:855-864.). Ethical approval for animal studies was obtained from the Institutional Animal Care and Use Committee at the University of Alabama (Birmingham, Alabama) and from Corixa Corporation.

### Example 10

### Specificity of CD4⁺ T cell stimulation

APCs were BALB/c spleen cells that were pulsed for 24 hours with nothing, OVA peptide at 2 µg/ml, CBir1 at 100 µg/ml, or Fla-X at 100 µg/ml, alone or in combinations as shown in Table 2. These APCs were washed and irradiated with 3,000 rads prior to culture. CD4⁺ T cells were isolated from DO11.10 mice and were cultured at a density of 1 x 10⁵ with 4 x 10⁵ prepulsed APCs. [³H]TdR was added at day 3 of culture and the cells were harvested after 16 hours.

### Example 11

### Adoptive transfer

CD4⁺ T cells were cultured with cecal bacterial antigen-pulsed and irradiated C3H/HeJ splenic cells in complete medium at 37°C for 4 days in 5% CO₂ air before being transferred intravenously into C3H/HeSnJ *scid*/*scid* recipients. Three months later, the recipients were killed and then the cecum and the proximal, medial, and distal portions of the colon were fixed in formalin. Fixed tissues were embedded in paraffin, and sections were stained with hematoxylin and eosin for histological examination. All slides were "read" by an experienced pathologist (A. Lazenby, Department of Pathology, University of Alabama at Birmingham) without knowledge of their origin.

### Example 12

### Human subjects

Serum samples from 212 subjects (50 UC patients, 100 CD patients, 22 DCs, and 40 NCs) were obtained from the serum archive of the Cedars-Sinai IBD Research Center. Sera were produced from standard phlebotomy blood specimens and were given an "anonymous" number code, divided into aliquots, and stored at -80°C until use. The UC and CD patient specimens were obtained from a genetic case-control study (Toyoda H, et al. Distinct associations of HLA class II genes with inflammatory bowel disease. Gastroenterology. 1993;104:741-748. Yang H-Y, et al. Ulcerative colitis: a genetically heterogeneous disorder defined by genetic (HLA class II) and subclinical (antineutrophil cytoplasmic antibodies) markers. J. Clin. Invest. 1993;92:1080-1084.). Each patient's diagnosis was confirmed by clinical history, endoscopic and radiologic examination, and histopathology findings. The NC group is a collection of environmental controls that contain sera from individuals with no symptoms/signs of disease (i.e., spouses). DC samples include sera from patients with presumed infectious enteritis (stool culture negative for specific pathogens), blastocystis, celiac disease, collagenous colitis, irritable bowel syndrome, radiation proctitis, and acute schistosomiasis. The UC group includes both pANCA-positive and -negative specimens, while the CD group contains samples that are marker-negative, ASCA+; I2+; OmpC+ (I2-positive); OmpC+, I2+, and ASCA+; and pANCA+. Ethical approval for human studies was obtained from the institution review board at Cedars-Sinai Medical Center.

### Example 13

### Nucleotide sequence accession numbers

The nucleotide sequence data for the flagellins CBir1 and Fla-X have been assigned GenBank accession numbers AY551005 and AY551006, respectively.

### Example 14

### Seroreactivity in mice is directed mainly against a specific group of flagellins

Serologic expression cloning resulted in 55 clones that were sequenced and identified. Using the basic local alignment search tool to search the GenBank databases demonstrated that 15 (26.8%) of these clones were flagellin-like sequences. None of the sequences directly matched any flagellin in the GenBank database, and all flagellin sequences identified represented unique clones. Given the average insert size of 0.8 kb in the library, no full-length flagellin clones were identified. However, all of the flagellin clones contained sequences derived from the conserved NH₂ terminus, with varying amounts of the hypervariable central domain, and only two clones contained partial sequence from the conserved carboxy domain. Sequences from the 15 flagellin clones identified (CBir1-CBir15) were aligned at the protein level to flagellin sequences available in the public domain using the Clustal program in DNAStar. As shown in Figure 1B, these flagellins are most closely related to flagellins from *Butyrivibrio*, *Roseburia, Thermotoga,* and *Clostridium* species and appear to align, by similarity, in the *Clostridium* subphylum cluster XIVa of Gram-positive bacteria. Sequences from the remaining 40 clones (see Table 1) were also unique and were either related to known proteins (33 clones) or without significant homology to known proteins (7 clones).

**Table 1. Identity of serological expression clones**

| **No. Clones** | **Homology** |
|---|---|
| 15 | Flagellins |
| 6 | Ribosomal proteins |
| 4 | Elongation factors |
| 3 | Chemotaxis proteins |
| 2 | Transcription regulators |
| 1 | Motility protein A |
| 1 | Surface Ag BspA |
| 1 | ABC transport protein |
| 1 | ParB protein |
| 1 | Multimeric flavodoxin WrbA |
| 1 | Toprim domain protein |
| 1 | dnaA |
| 1 | Two-component sensor protein |
| 10 | Enzymes |
| 7 | Novel/hypothetical |

| | |
|---|---|
| Number of clones with a similar homology (No. clones). BspA, bacteroides surface protein A; ParB, chromosome partitioning protein B; WrbA, tryptophan-repressor-binding protein A; dnaA, chromosome replication initiator A. | |

Because of strong serum antibody reactivity to one particular flagellin clone, called CBir1, it was cloned and expressed its full-length gene. During this effort, the inventors also cloned a second, highly homologous and reactive flagellin (83.5% similarity to CBir1 at the NH₂ conserved domain) and refer to it here as Fla-X (Figure 2B). Recombinant proteins representing full-length sequence and NH₂ and carboxy fragments of both CBir1 and Fla-X were subsequently expressed in *E. coli* with a six-histidine tag to aid in protein purification (Figure 2, C and D, respectively).

### Example 15

### Antibody reactivity to flagellin directed against the NH₂ terminus is of the IgG2a subclass and correlates with disease.

Western blot analyses using these purified recombinant flagellins and fragments with sera from the diseased C3H/HeJBir mice demonstrated the strong reactivity to flagellin, predominantly to the NH₂-terminal fragments (Figure 3A). Little or no antibody reactivity was seen to the carboxy-terminal CBir1 or Fla-X recombinant fragments in the sera tested (Figure 3). This selective reactivity to the NH₂ domain is consistent with the presence of an NH₂ domain in all flagellin clones identified in the initial serological screen (Figure 1). In addition, strong reactivity to both flagellins was seen using sera from two additional experimental models of colitis: *mdr1a*^{*-*/*-*} mice (Figure 3B) and B6.IL-10^{-/-} mice. These last two models are on strains with different haplotypes from each other (H-2^{s} and H-2^{b}, respectively) and from the C3H/HeJBir strain (H-2^{k}), and the sera were obtained from mice from geographically different mouse facilities. In addition, these additional colitic strains have very different mechanisms underlying the genetic predisposition to develop IBD; that is, epithelial barrier dysfunction in the *mdr1a*^{*-*/*-*} mice and a defect in regulatory T cells in the B6.IL-10^{-/-} mice. Little or no reactivity was seen to CBir1 or Fla-X in noncolitic mouse serum from control MHC haplotype-matched noncolitic mice (Figure 3, A and B). Interestingly, the inventors also saw a similar pattern of reactivity to the NH₂ termini of both CBir1 and Fla-X with a serum pool from patients with CD (Figure 3C).

In order to generate more quantitative data with multiple IBD models at various time points in the course of disease, an antibody subclass ELISA against full-length or fragments of CBir1 or Fla-X was developed (Figure 2, C and D). This assay confirmed Western blot data showing that the antibody reactivity observed was predominantly to the NH₂ terminus (data not shown) and of the IgG2a subclass. High titers of anti-flagellin antibody were seen in the four genetically distinct models of IBD tested (colitic mice: C3H/HeJBir [Figure 4], *mdr1a*^{*-*/*-*} [Figure 4], BALB/c.IL-10^{-/-} [not shown] and B6.IL-10^{-/-} [not shown]), while minimal to no reactivity was seen in serum from the H-2-matched, control, noncolitic mouse strains. Given the nonuniform incidence of colitis in the *mdr1a*^{*-*/*-*} colony at varying time points, the inventors randomly chose 23 animals in the colony and assigned quantitative histopathological scores using a scale (from 0 to 60) that incorporates both the degree and extent of inflammation in the large intestine (Burich A, et al. Helicobacter-induced inflammatory bowel disease in IL-10- and T cell-deficient mice. Am. J. Physiol. Gastrointest. Liver Physiol. 2001;281:G764-G778.). The inventors measured antibodies against Fla-X and CBir1 by ELISA in a "blinded" manner using sera from these animals and found that an increased titer of anti-flagellin IgG correlated positively with worsening IBD histopathology (*r*= +0.758 and +0.719, respectively; Figure 5). Weak correlations were found between antibody and mouse age (*r*= +0.325) and between colitis score and mouse age (*r*= +0.372).

### Example 16

### Anti-CBir1 reactivity in CD patient sera but not normal or UC patient sera

Subsequently, a large panel of sera from controls and patients with IBD for reactivity against CBir1 and Fla-X using antigen-specific ELISAs was tested. It was found that a significantly higher level of serum anti-CBir1/Fla-X flagellin in CD patients than in NCs, disease controls (DCs), and UC patients (Figure 6A). It should be noted that more than 50% of the UC sera were from patients with a modified Truelove and Witts severity index greater than 7, indicating moderate to active disease. The observation of serum responses to flagellins CBir1 and Fla-X in a group of CD patients, but not UC patients (Figure 6A), highlights the possibility that anti-flagellin responses may be valuable in the diagnosis of IBD, in particular with regards to the more precise discrimination between UC and CD, and the definition of CD patient subsets.

Reactivity to the *Salmonella muenchen* flagellin (which is highly similar to the flagellin of *Eschericia coli* [84-91% at the conserved NH₂ end]), however, showed no significant correlation to disease (Figure 6B). As shown in Figure 6B, the mean values for the *anti-Salmonella* response were nearly identical in the control, CD, and UC populations. In all populations, there appeared to be a minority of samples without significant reactivity and a majority of samples that were "positive." While these data may reflect the random exposure to *Salmonella* in humans due to prior infection (possibly subclinical) or a cross-reactivity to an undefined but closely related flagellin (probably from the *Enterobacteriaceae* family), it is clear that the serological response to the *Salmonella* flagellin molecule does not correlate with IBD. Similarly, there was no correlation between reactivity to *Salmonella* flagellin and colitis in the C3H/HeJBir or *mdr1a*^{*-*/*-*} strains compared with the MHC haplotype-matched controls.

Marked reactivity against flagellin is seen at the T cell level, and flagellin-specific T cells are able to induce colitis when adoptively transferred. Given the strong IgG2a bias seen in the antibody response in the mouse IBD strains, and while not wishing to be bound by any particular theory, it is believed that flagellin-specific Th1 T cells would be present in mice with IBD. To address this possibility, CD4⁺ T cells from pooled spleens and mesenteric lymph nodes from colitic *mdr1a*^{*-*/*-*}, C3H/HeJBir, and C3H/HeJ.IL-10^{-/-} mice (and haplotype-matched, noncolitic FVB and C3H/HeJ mice) were purified and tested the cells for reactivity against purified CBir1 and Fla-X in vitro in the presence of antigen-presenting cells (APCs). CD4⁺ T cells from the colitic *mdr1a*^{*-*/*-*}, C3H/HeJBir, and C3H/HeJ.IL-10^{-/-} mice, but not from age-matched control FVB or C3H/HeJ mice raised in the same mouse facility, responded to CBir1, as assessed by proliferation (Figure 7). It was possible that the responses seen were due to the fact that the flagellin molecule was nonspecifically activating the cultured T cells via TLR5 or TLR4 activation (through endotoxin contamination of the recombinant protein). This possibility was excluded by the lack of stimulation in both the noncolitic T cell cultures (Figure 7) and in an independent T cell culture system that showed no influence of Fla-X or CBir1 on the ovalbumin-specific proliferation of CD4⁺ T cells from DO11.10 ovalbumin-specific T cell receptor-transgenic animals (Table 2).

**Table 2. Specificity of T cell activation**

| **CD4⁺ T cells** | **APC** | **Antigen** | **Mean cpm ± SD** |
|---|---|---|---|
| DO11.10 | None | None | 232 ± 48 |
| DO11.10 | + | None | 223 ± 37 |
| DO11.10 | + | OVA | 63,104 ± 6,379 |
| DO11.10 | + | CBir1 | 1,036 ± 150 |
| DO11.10 | + | Fla-X | 876 ± 1,045 |
| DO11.10 | + | OVA + CBir1 | 58,831 ± 4,684 |
| DO11.10 | + | OVA + Fla-X | 64,300 ± 1,314 |

| | | | |
|---|---|---|---|
| OVA-specific T cell line DO11.10 proliferates specifically in the presence of OVA peptide, but not nonspecifically in the presence of recombinant proteins CBir1 or Fla-X. | | | |

It has been previously shown that a T cell line specific for cecal bacterial protein/antigen (CBA), but not CD4⁺ T cells polyclonally activated by anti-CD3, could induce mucosal inflammation when adoptively transferred into H-2-matched immunodeficient *scid*/*scid* mice (Cong Y, et al. CD4+ T cells reactive to enteric bacterial antigens in spontaneously colitic C3H/HeJBir mice: increased T helper cell Type 1 response and ability to transfer disease. J. Exp. Med. 1998;187:855-864.). To address the potentially pathogenic role of flagellin-specific T cells in the initiation of mucosal inflammation, a CD4⁺ T cell line reactive with CBir1 flagellin from C3H/HeJBir mice was generated by repeated stimulation with antigen and APCs. This CD4⁺ T cell line strongly responded to CBir1 but not to Fla-X or a variety of other microbial, food, and epithelial antigens (Figure 8). These CBir1-specific CD4⁺ T cells were adoptively transferred into C3H/HeJ-*scid*/*scid* recipients. Control SCID mice received anti-CD3-activated CD4⁺ T cells as a negative control or a CD4⁺ T cell line reactive to CBA as a positive control. Quantitative histopathological scores were assigned at 8 weeks after transfer (Figure 9A). The CBir1-specific CD4⁺ T cell line induced colitis in all recipients of an intensity that was similar to or greater than that induced by the CBA-specific CD4⁺ T cell line, whereas none of the recipients given anti-CD3-activated C3H/HeJBir CD4⁺ T cells developed disease (representative histology is shown in Figure 9B).

### Example 17

### Human subjects

Serum samples from 484 subjects (40 normal controls (NC), 21 disease controls (DC), 50 UC patients, and 373 CD patients) were selected from the serum archive of the Cedars-Sinai IBD Research Center. All research related activities were approved by the Cedars-Sinai Medical Center, Institutional Review Board. Diagnosis for each patient was based on standard endoscopic, histologic, and radiographic features. The normal control (NC) group is a collection of environmental controls that contain sera from individuals with no symptoms/signs of disease (i.e. spouses of patients). Disease controls (DC) include sera from patients with presumed infectious enteritis (stool culture negative for specific pathogens), blastocystis, celiac disease, collagenous colitis, irritable bowel syndrome, radiation proctitis, and acute schistosomiasis. For UC, groups chosen were pANCA- (n=25, seronegative) and pANCA⁺ (n=25, pANCA EU > 45, no other antibody reactivity present). For CD, two cohorts were chosen: Cohort 1 (Lodes MJ, Cong Y, Elson CO, Mohamath R, Landers CJ, Targan SR, Fort M, Hershberg RM. Bacterial flagellin is a dominant antigen in Crohn disease. J Clin Invest 2004;113:1296-306.) (n=100) was comprised of patients with select antibody expression to test CBir-1's specificity for CD and its relationship with other CD associated antibodies, Cohort 2 (n=303) was unbiased, previously well clinically and serologically characterized (Mow WS, Vasiliauskas EA, Lin YC, Fleshner PR, Papadakis KA, Taylor KD, Landers CJ, Abreu-Martin MT, Rotter JI, Yang H, Targan SR. Association of antibody responses to microbial antigens and complications of small bowel Crohn's disease. Gastroenterology 2004;126:414-24.), with an overlap of 30 patients between the two. Within Cohort 1, groups chosen were seronegative (n=40), ASCA⁺ (n=15, IgG ASCA EU>40, IgA ASCA EU>45, and no other antibody reactivity present), I2⁺ (n=15, anti-I2 EU>40, and no other antibody reactivity present), I2⁺/OmpC⁺ (n=15, OmpC EU>30, and anti-I2 reactivity present), I2⁺/OmpC⁺/ASCA⁺ (n=15, anti-12, anti-OmpC, IgG and IgA ASCA all positive, but no ANCA reactivity allowed), and pANCA⁺ (n=25, ANCA EU>35, no other antibody reactivity present). Cohort 2 was used for determining antibody groups as well as for phenotype analysis using definitions of clinical subgroup previously reported (Mow WS, Vasiliauskas EA, Lin YC, Fleshner PR, Papadakis KA, Taylor KD, Landers CJ, Abreu-Martin MT, Rotter JI, Yang H, Targan SR. Association of antibody responses to microbial antigens and complications of small bowel Crohn's disease. Gastroenterology 2004;126:414-24.). Serum samples from 44 CD patients diagnosed as above were analyzed for changes in antibody expression. Twenty of these patients were under treatment with infliximab and had experienced a CD Activity Index (CDAI) change of at least 70 (mean=Δ181) at time points at least 4 months apart with serum drawn at both times. The other 24 patients were drawn at the time of surgery and once at least 6 months following surgery.

### Example 18

### ELISA

ELISA analysis of anti-CBir1 was performed as described in Lodes, et al. (Lodes MJ, Cong Y, Elson CO, Mohamath R, Landers CJ, Targan SR, Fort M, Hershberg RM. Bacterial flagellin is a dominant antigen in Crohn disease. J Clin Invest 2004;113:1296-306.) but using NH₂-terminal fragment of of CBir1 (147aa) without knowledge of diagnosis or other serology results. Briefly, ELISA plates were coated overnight with 100ng/well of CBir1, then blocked with 1 % BSA in PBS for 2 hours. Plates were washed and serum was added at a 1:200 dilution in 1% BSA-PBS for a 30 minute incubation. After washing, horseradish peroxidase conjugated anti-human IgG at a 1:10,000 dilution was added and incubated for 30 minutes. After another wash, the plates were incubated with tetramethylbenzidine substrate for 15 minutes. The reaction was stopped with 1 N sulfuric acid and read at 450nm. Positive was defined as the mean + 2 SD of the healthy controls. For Cohort 2 and the longitudinal cohorts and phenotype cohorts, this assay was modified to be more similar to the ANCA, OmpC and I2 protocols: alkaline phosphatase was substituted as the secondary conjugate and incubated for 1 hour followed by paranitrophenyl phosphate as substrate for 30 minutes.

### Example 19

### Statistical Analysis

Differences between disease groups were tested with non-parametric (Wilcoxon signed rank) statistics for quantitative levels. To determine the associations between antibody responses (positivity) toward microbial antigens, auto-antigens, and disease phenotype characteristics, univariate analyses utilizing χ² tests were performed. The Cochran-Armitage test for trend was utilized to test if there is a linear trend in the proportion of patients with positive anti-CBir 1 expression as the number of antibody responses increased. A p-value (p trend) <=0.05 suggests that the linear trend is statistically significant. A stratified Cochran-Mantel-Haenszel test was used to determine the association between anti-CBir1 and disease phenotypes. Multivariate analysis with logistic regression modeling was also performed to determine the primary associations among qualitative serological responses with disease phenotypes. All statistic tests were preformed using Statistical Analysis Software (Version 8.02; SAS Institute, Inc., Cary, NC).

### Example 20

### Serum reactivity to CBir1 defines a subset of patients with Crohn's disease

Serologic expression cloning of a cecal bacterial antigen phage library identified the flagellin, CBir1, as an immunodominant antigen recognized by colitic mice and by approximately half of patients with CD. Serum from two separate cohorts was used to investigate subgroups of CD patients. Cohort 1 consisted of sera from 100 CD patients selected on the basis of antibody profile. Newly tested sera from a group of 303 unselected patients that were studied and reported on in Mow et al (Mow WS, Vasiliauskas EA, Lin YC, Fleshner PR, Papadakis KA, Taylor KD, Landers CJ, Abreu-Martin MT, Rotter JI, Yang H, Targan SR. Association of antibody responses to microbial antigens and complications of small bowel Crohn's disease. Gastroenterology 2004;126:414-24.) comprised Cohort 2. For antigen, the amino domain of CBir1 flagellin was used because most of the IgG reactivity was to this region of the molecule. In addition, this form of CBir1 had a lower baseline reactivity among inflammatory controls, patients with UC or CD, and healthy control subjects, compared to the full length construct. As shown in Figure 10, 50% of CD patients from the Cohort 1 had serologic responses to this CBir1 construct, as compared to very low numbers and low levels of response among inflammatory controls, patients with UC, or healthy control subjects. Among the control subjects who did respond to CBir1, the level of response was much lower than that of the patients with CD. In the unselected cohort, Cohort 2, 55% (167 of 303) of sera were positive for antibodies to CBir1. Approximately half of CD patients, whether selected serologically or not, are reactive to CBir1.

### Example 21

### Levels of antibodies to CBir1 do not correlate with disease activity

As had been done with the previously defined CD-related antigens (12/OmpC, oligomannan; Landers CJ, Cohavy O, Misra R, Yang H, Lin YC, Braun J, Targan SR. Selected loss of tolerance evidenced by Crohn's disease-associated immune responses to auto- and microbial antigens. Gastroenterology 2002;123:689-99. Mow WS, Vasiliauskas EA, Lin YC, Fleshner PR, Papadakis KA, Taylor KD, Landers CJ, Abreu-Martin MT, Rotter JI, Yang H, Targan SR. Association of antibody responses to microbial antigens and complications of small bowel Crohn's disease. Gastroenterology 2004;126:414-24.), determining whether the level of anti-CBir1 expression changed in association with disease activity was sought. Serum samples from medically-resistant patients with CD, who were undergoing surgical removal of active disease were collected. Samples were taken again 6 months post-operatively and analyzed for differences in response. In general, there was very little change before and 6 months post surgery, when patients were in clinical and endoscopic remission (Figure 11 A). The same analysis was performed before, and 4 months after, treatment of CD with infliximab (Figure 11B, C). Among patients who achieved complete remission as evidenced by mucosal changes and healing, similar stability in anti-CBir1 expression is seen (Figure 11B, C). These findings are consistent with antibody responses to other microbial antigens (Landers CJ, Cohavy O, Misra R, Yang H, Lin YC, Braun J, Targan SR. Selected loss of tolerance evidenced by Crohn's disease-associated immune responses to auto- and microbial antigens. Gastroenterology 2002;123:689-99. Mow WS, Vasiliauskas EA, Lin YC, Fleshner PR, Papadakis KA, Taylor KD, Landers CJ, Abreu-Martin MT, Rotter JI, Yang H, Targan SR. Association of antibody responses to microbial antigens and complications of small bowel Crohn's disease. Gastroenterology 2004;126:414-24.).

### Example 22

### Antibody response to CBir1 and other Crohn's disease-associated anti-microbial immune responses

To determine the relationship of expression and level of anti-CBir1 expression to the previously defined antibodies to microbial antigens (anti-12, anti-OmpC, ASCA), multiple logistic regression analysis with Cohort 1 was used, it was found that anti-CBir1 relates independently to CD when controlled for anti-I2, anti-OmpC and ASCA (p<0.001). In addition, there is no relationship between the *level of* response to CBir1 and *any one* of the other four antibodies (Figure 12A-D). Thus, reactivity to CBir1 defines another potentially pathophysiogically distinct subgroup of CD.

As previously described by Landers et al (Landers CJ, Cohavy O, Misra R, Yang H, Lin YC, Braun J, Targan SR. Selected loss of tolerance evidenced by Crohn's disease-associated immune responses to auto- and microbial antigens. Gastroenterology 2002;123:689-99.), homogeneous groups of CD patients based on selective antibody responses to specific microbial antigens and associated clinical features were defined. Thus far, the largest subgroup of CD has been stratified based on expression of ASCA. Among patients selected for study based on their antibody profiles (Cohort 1), anti-CBir1 is expressed in bothASCA- negative (46%) and ASCA+ (60%) CD patients (Figure 13). Anti-CBir1 is also expressed by patients who do not react to ASCA, OmpC, I2, or ANCA (40%) as well as those who express ASCA exclusively (33%) and anti-I2 exclusively (40%) (Figure 14). Anti-CBir1 expression and magnitude increases among patients reactive to both I2 and OmpC (60%), and increases more among those patients reactive to I2, OmpC and oligomannan (87%) (Figure 14). These results were confirmed in Cohort 2, in which the inventors found anti-CBir1 expression in 46% (66/144) of ASCA-, 64% (102/159) of ASCA⁺, and 38% (23/61) of seronegative CD patients. The frequency of anti-CBir1 expression increases as the number of positive antibody responses increases in patients with 0, 1, 2, and 3 antigens (Figure 15, p<0.001). Thus, in both Cohort 1 and Cohort 2, anti-CBir1 expression is highest in sera from patients who react to all three other antigens, but is also found along with any other combination of 1, 2, or 3 antibody responses.

### Example 23

### Antibodies to CBir1 and pANCA Expression

A small percentage of CD patients are solely pANCA-positive. pANCA is associated with ulcerative colitis, and in CD, pANCA marks for left-sided disease with ulcerative colitis-like features (Vasiliauskas EA, Plevy SE, Landers CJ, Binder SW, Ferguson DM, Yang H, Rotter JI, Vidrich A, Targan SR. Perinuclear antineutrophil cytoplasmic antibodies in patients with Crohn's disease define a clinical subgroup. Gastroenterology 1996;110:1810-9. Vasiliauskas EA, Kam LY, Karp LC, Gaiennie J, Yang H, Targan SR. Marker antibody expression stratifies Crohn's disease into immunologically homogeneous subgroups with distinct clinical characteristics. Gut 2000;47:487-96. Esters N, Vermeire S, Joossens S, Noman M, Louis E, Belaiche J, De Vos M, Van Gossum A, Pescatore P, Fiasse R, Pelckmans P, Reynaert H, Poulain D, Bossuyt X, Rutgeerts P. Serological markers for prediction of response to anti-tumor necrosis factor treatment in Crohn's disease. Am J Gastroenterol 2002;97:1458-62. Peeters M, Joossens S, Vermeire S, Vlietinck R, Bossuyt X, Rutgeerts P. Diagnostic value of anti-Saccharomyces cerevisiae and antineutrophil cytoplasmic autoantibodies in inflammatory bowel disease. Am J Gastroenterol 2001;96:730-4.). pANCA does not differentiate between UC and UC-like CD. Determine whether anti-CBir1 expression had any bearing on this subgroup was sought. Of the pANCA⁺ patients with CD, 40-44% (Cohort 2 and Cohort 1, respectively) expressed anti CBir1 and none of other antibodies v. only 4% in pANCA⁺ ulcerative colitis (Figure 16). This difference stratifies another subgroup of CD with a potential pathophysiologically unique disease mechanism.

### Example 24

### Crohn's Disease Phenotypic Associations with Anti-CBir1 Expression

It was previously determined that antibody responses to the microbial antigens, OmpC, I2, oligomannan and neutrophil nuclear antigen(s) is associated with anatomical location as well as disease expression (Vasiliauskas EA, Plevy SE, Landers CJ, Binder SW, Ferguson DM, Yang H, Rotter JI, Vidrich A, Targan SR. Perinuclear antineutrophil cytoplasmic antibodies in patients with Crohn's disease define a clinical subgroup. Gastroenterology 1996;110:1810-9. Vasiliauskas EA, Kam LY, Karp LC, Gaiennie J, Yang H, Targan SR. Marker antibody expression stratifies Crohn's disease into immunologically homogeneous subgroups with distinct clinical characteristics. Gut 2000;47:487-96. Mow WS, Vasiliauskas EA, Lin YC, Fleshner PR, Papadakis KA, Taylor KD, Landers CJ, Abreu-Martin MT, Rotter JI, Yang H, Targan SR. Association of antibody responses to microbial antigens and complications of small bowel Crohn's disease. Gastroenterology 2004;126:414-24. Arnott IDR, Landers CJ, Nimmo EJ, Drummond HE, Targan SR, Satsangi J. Reactivity to microbial components in Crohn's disease is associated with severity and progression. Am J Gastroenterol 2004;99:2376-84.). Anti-CBir1 expression appears to be independently associated with CD. Therefore, to determine whether a clinical phenotype is independently associated with anti-CBir1 expression, the serotypically- and phenotypically-defined Cohort 2 was used to assess the overall and specific phenotypes associated with anti-CBir1 expression. It was found that 61 % of patients with complicated (internal penetrating, fibrostenosing disease features, and those with or without history of surgery) were anti-CBir1⁺, compared to 42% of patients with inflammatory-only CD (p=0.002). Anti-CBir1 expression was positively associated with small bowel disease, fibrostenosing and internal penetrating disease (see Table 3), regardless of the presence of antibodies to 1, 2, or all 3 other antigens. Unlike some of the other antibody responses, anti-CBir1 expression was neither associated with small bowel surgery, nor was it negatively associated with the UC-like CD population. To further assess the independent relationship of anti-CBir1 expression to CD phenotypes, the inventors performed a multivariate logistic regression model analysis with the four CD-associated antibodies. The results in Table 4 show that anti-CBir1 expression is independently associated with small bowel, internal penetrating and fibrostenosing disease. Consistent with this finding is the lower frequency of anti-CBir1 expression in the cohort of patients treated with infliximab (30%, Figure 11), among whom internal penetrating and fibrostenosing disease features would not be prevalent. Thus, anti-CBir1 expression is independently associated with CD, but also selects for a specific phenotype.

**Table 3: Phenotypic Associations with anti-CBir1**

| **Phenotype** | **OR** | **CI** | ***P value** |
|---|---|---|---|
| Small bowel disease | 2.16 | 1.22-3.30 | 0.009 |
| Fibrostenosis | 1.71 | 1.05-2.80 | 0.03 |
| Internal perforating disease | 2.01 | 1.22-3.30 | 0.006 |

| | | | |
|---|---|---|---|
| OR=odds ratio CI=confidence interval *stratified Cochran-Mantel-Haenszel | | | |

**Table 4: Clinical Features: Results of Multivariate Logistic Regression**

| | Small Bowel Disease | Fibrostenosis | Internal Perforating | Small Bowel Surgery | UC-Like |
|---|---|---|---|---|---|
| Anti-CBir1 | 0.0099 | 0.0402 | 0.0093 | NS | NS |
| ASCA | 0.0194 | <0.0001 | 0.0009 | 0.0002 | <0.000 1 |
| Anti-OmpC | NS | NS | 0.01 | NS | NS |
| Anti-I2 | NS | 0.0236 | NS | 0.0077 | NS |

While the description above refers to particular embodiments of the present invention, it should be readily apparent to people of ordinary skill in the art that a number of modifications may be made without departing from the spirit thereof. The accompanying claims are intended to cover such modifications as would fall within the true spirit and scope of the invention. The presently disclosed embodiments are, therefore, to be considered in all respects as illustrative and not restrictive, the scope of the invention being indicated by the appended claims rather than the foregoing description. All changes that come within the meaning of and range of equivalency of the claims are intended to be embraced therein.
Further embodiments include:
**(1)** A method of diagnosing Crohn's disease in a mammal, comprising: obtaining a sample from the mammal; and determining the presence of anti-CBir1 expression in the sample, wherein the presence of anti-CBir1 expression indicates that the mammal has Crohn's disease.
   The Crohn's disease is preferably Crohn's disease with characteristics of small bowel disease, internal perforating disease, and/or fibrostenosing disease and the presence of the RNA sequence or a fragment of an RNA sequence that encodes the anti-CBir1 antibody may be determined using a technique selected from the group consisting of Northern blot analysis and reverse transcription-polymerase chain reaction (RT-PCR).
   The presence of expression may involve determining the presence of an RNA sequence or a fragment of an RNA sequence that encodes an anti-CBir1 antibody in the sample obtained from the mammal.
   Determination of the presence of anti-CBir1 expression in a mammal may comprise: determining the presence of an anti-CBir1 antibody in the sample obtained from the mammal. The anti-CBir1 antibody may be IgG anti-CBir1. The presence of the anti- CBir1 antibody may comprise using a technique selected from the group consisting of enzyme-linked immunosorbent assay (ELISA), sodium dodecyl sulfate polyacrylamide gel electrophoresis (SDS-PAGE), Western blot analysis and mass spectrometric analysis. The presence of the anti- CBir1 antibody may be determined by contacting the sample from the mammal with a CBir1 flagellin antigen, or immunoreactive fragment thereof, under conditions suitable to form a complex of CBir1 flagellin antigen or immunoreactive fragment thereof, and an antibody against the CBir1 flagellin antigen; contacting the complex with a labeled secondary antibody; and detecting the presence of the complex.
(2) A method for treating Crohn's disease in a mammal, comprising diagnosing Crown's disease, wherein diagnosing Crohn's disease comprises: obtaining a sample from the mammal, and determining the presence of anti-CBir1 expression in the sample obtained from the mammal, wherein the presence of anti-CBir1 expression indicates that the mammal has Crohn's disease; and using an antigen-directed therapy on the mammal to treat the Crohn's disease.
   The antigen-directed therapy may target a CBir1 flagellin antigen, an immunoreactive fragment thereof, or combinations thereof.
(3) A kit for diagnosing Crohn's disease or a subtype of Crohn's disease in a mammal, comprising:
   a quantity of CBiM flagellin antigen, or an immunoreactive fragment thereof; and
   instructions for diagnosing Crohn's disease or a subtype of Crohn's disease in a mammal.

The instructions for diagnosing Crohn's disease or a subtype of Crohn's disease may comprise instructions for determining the presence of an anti-CBir1 antibody. Such instructions for determining the presence of the anti-CBir1 antibody may comprise: instructions for contacting a sample obtained from a mammal with a CBir1 flagellin antigen, or immunoreactive fragment thereof, under conditions suitable to form a complex of CBir1 flagellin antigen, or immunoreactive fragment thereof, and an antibody against the CBir1 flagellin antigen; instructions for contacting the complex with a labeled secondary antibody; and instructions for detecting the presence of the complex.

## Claims

1. A method of diagnosing a subtype of Crohn's disease in a mammal with Crohn's disease, comprising:
determining the presence of anti-CBir1 expression in a sample obtained from the mammal; and
determining the presence of a perinuclear anti-neutrophil cytoplasmic antibody (pANCA) in the sample obtained from the mammal,
wherein the presence of anti-CBir1 expression and the pANCA indicates the subtype of Crohn's disease with features of colitic disease and/or colitic and small bowel disease.

2. The method of claim 1, wherein determining the presence of anti-CBir1 expression comprises:
determining the presence of an RNA sequence or a fragment of an RNA sequence that encodes an anti-CBir1 antibody from the sample obtained from the mammal.

3. The method of claim 2, wherein determining the presence of the RNA sequence or the fragment of an RNA sequence that encodes the anti- CBir1 antibody comprises using a technique selected from the group consisting of Northern blot analysis and reverse transcription-polymerase chain reaction (RT-PCR).

4. The method of claim 1, wherein determining anti-CBir1 expression in a mammal comprises:
determining the presence of an anti-CBir1 antibody in the sample obtained from the mammal.

5. The method of claim 4, wherein the anti-CBirl antibody is IgG anti-CBir1.

6. The method of claim 4, wherein determining the presence of the anti- Cbir1 antibody comprises using a technique selected from the group consisting of ELISA, SDS-PAGE, Western blot analysis and mass spectrometric analysis.

7. The method of claim 1, wherein determining the presence of pANCA comprises using a technique selected from the group consisting of ELISA, SDS-PAGE, Western blot analysis and mass spectrometric analysis.

## Patentansprüche

1. Verfahren zum Diagnostizieren eines Subtyps von Morbus Crohn bei einem Säugetier mit Morbus Crohn, umfassend:
Bestimmen des Vorliegens einer Anti-CBir1-Expression in einer von dem Säugetier erhaltenen Probe; und
Bestimmen des Vorliegens eines perinukleären anti-neutrophilen zytoplasmatischen Antikörpers (pANCA) in der von dem Säugetier erhaltenen Probe,
wobei das Vorliegen der Anti-CBir1-Expression und des pANCA den Subtyp von Morbus Crohn mit Merkmalen von Colitis und/oder Colitis und Dünndarmerkrankung angibt.

2. Verfahren nach Anspruch 1, wobei das Bestimmen des Vorliegens der Anti-CBir1-Expression Folgendes umfasst:
Bestimmen des Vorliegens einer RNA-Sequenz oder eines Fragments einer RNA-Sequenz, die für einen Anti-CBir1-Antikörper aus der von dem Säugetier erhaltenen Probe codiert.

3. Verfahren nach Anspruch 2, wobei das Bestimmen des Vorliegens der RNA-Sequenz oder des Fragments einer RNA-Sequenz, die für den Anti-CBir1-Antikörper codiert, die Anwendung einer Technik umfasst, die aus der Gruppe ausgewählt ist, die aus Northern-Blot-Analyse und reverser Transkription-Polymerasekettenreaktion (RT-PCR) besteht.

4. Verfahren nach Anspruch 1, wobei das Bestimmen der Anti-CBir1-Expression bei einem Säugetier Folgendes umfasst:
Bestimmen des Vorliegens eines Anti-CBirl-Antikörpers in der von dem Säugetier erhaltenen Probe.

5. Verfahren nach Anspruch 4, wobei der Anti-CBir1-Antikörper IgG-Anti-CBir1 ist.

6. Verfahren nach Anspruch 4, wobei das Bestimmen des Vorliegens des Anti-CBirl-Antikörpers die Anwendung einer Technik umfasst, die aus der Gruppe ausgewählt ist, die aus ELISA, SDS-PAGE, Western-Blot-Analyse und massenspektrometrischer Analyse besteht.

7. Verfahren nach Anspruch 1, wobei das Bestimmen des Vorliegens des pANCA die Anwendung einer Technik umfasst, die aus der Gruppe ausgewählt ist, die aus ELISA, SDS-PAGE, Western-Blot-Analyse und massenspektrometrischer Analyse besteht.

## Revendications

1. Procédé de diagnostic d'un sous-type de la maladie de Crohn chez un mammifère atteint de la maladie de Crohn consistant à :
déterminer la présence de l'expression anti-CBir1 dans un échantillon prélevé sur le mammifère ; et
déterminer la présence d'un anticorps périnucléaire anti-cytoplasme des neutrophiles (pANCA) dans l'échantillon prélevé sur le mammifère,
la présence de l'expression anti-CBir1 et le pANCA indiquant le sous-type de maladie de Crohn avec les caractéristiques de maladie colique et/ou de maladie colique et de l'intestin grêle.

2. Procédé de la revendication 1, dans lequel la détermination de la présence de l'expression anti-CBir1 consiste à :
déterminer la présence d'une séquence d'ARN ou d'un fragment d'une séquence d'ARN qui code un anticorps anti-CBir1 à partir de l'échantillon prélevé sur le mammifère.

3. Procédé de la revendication 2, dans lequel la détermination de la présence de la séquence d'ARN ou du fragment d'une séquence d'ARN qui code l'anticorps anti-CBir1 consiste à utiliser une technique choisie dans le groupe constitué par l'analyse par transfert Northern et la transcription inverse - réaction en chaîne à la polymérase (RT-PCR).

4. Procédé de la revendication 1, dans lequel la détermination de la présence de l'expression anti-CBir1 chez un mammifère consiste à :
déterminer la présence d'un anticorps anti-CBir1 dans l'échantillon prélevé sur le mammifère.

5. Procédé de la revendication 4, dans lequel l'anticorps anti-CBir1 est une IgG anti-CBir1.

6. Procédé de la revendication 4, dans lequel la détermination de la présence de l'anticorps anti-Cbir1 consiste à utiliser une technique choisie dans le groupe constitué par ELISA, SDS-PAGE, analyse par transfert Western et analyse par spectrométrie de masse.

7. Procédé de la revendication 1, dans lequel la détermination de la présence de pANCA consiste à utiliser une technique choisie dans le groupe constitué par ELISA, SDS-PAGE, analyse par transfert Western et analyse par spectrométrie de masse.


### SEQUENCE LISTING

<110> TARGAN, Stephan R.
   LANDERS, Carol J.
   YANG, Huiying
<120> METHODS FOR DIAGNOSIS AND TREATMENT OF
   CROHN'S DISEASE
<130> 67789-497
<150> 60/634,339 <151> 2004-12-08
<160> 15
<170> FastSEQ for windows version 4.0
<210> 1
   <211> 22
   <212> DNA
   <213> Artificial Sequence
<220>
   <223> Primer
<400> 1
   cacaatcaca acatctaccc ag 22
<210> 2
   <211> 27
   <212> DNA
   <213> Artificial Sequence
<220>
   <223> Primer
<400> 2
   ttactgtaag agctgaagta caccctg 27
<210> 3
   <211> 46
   <212> DNA
   <213> Artificial Sequence
<220>
   <223> Primer
<400> 3
   caattacata tgcatcacca tcaccatcac gtagtacagc acaatc 46
<210> 4
   <211> 38
   <212> DNA
   <213> Artificial Sequence
<220>
   <223> Primer
<400> 4
   atagactaag cttactgtaa gagctgaagt acaccctg 38
<210> 5
   <211> 53
   <212> DNA
   <213> Artificial Sequence
<220>
   <223> Primer
<400> 5
   caattacata tgcatcacca tcaccatcac gtagtacagc acaatttaca ggc 53
<210> 6
   <211> 38
   <212> DNA
   <213> Artificial Sequence
<220>
   <223> Primer
<400> 6
   atagactaag cttactgtaa gagctgaagt acaccctg 38
<210> 7
   <211> 37
   <212> DNA
   <213> Artificial Sequence
<220>
   <223> Primer
<400> 7
   atagactaag cttaagaaac cttcttgata gcgccag 37
<210> 8
   <211> 41
   <212> DNA
   <213> Artificial Sequence
<220>
   <223> Primer
<400> 8
   tagactgaat tctagtccat agcgtcaacg ttctttgtgt c 41
<210> 9
   <211> 53
   <212> DNA
   <213> Artificial Sequence
<220>
   <223> Primer
<400> 9
   caattacata tgcatcacca tcaccatcac aagatgaact tccatgtagg tgc 53
<210> 10
   <211> 46
   <212> DNA
   <213> Artificial Sequence
<220>
   <223> Primer
<400> 10
   caattacata tgcatcacca tcaccatcac gtagtacagc acaatc 46
<210> 11
   <211> 38
   <212> DNA
   <213> Artificial Sequence
<220>
   <223> Primer
<400> 11
   atagactaag cttactgtaa gagctgaagt acaccctg 38
<210> 12
   <211> 53
   <212> DNA
   <213> Artificial Sequence
<220>
   <223> Primer
<400> 12
   caattacata tgcatcacca tcaccatcac gtagtacagc acaatcttag age 53
<210> 13
   <211> 36
   <212> DNA
   <213> Artificial Sequence
<220>
   <223> Primer
<400> 13
   atagactaag cttagaggct gaaatcaatg tcctcg 36
<210> 14
   <211> 38
   <212> DNA
   <213> Artificial Sequence
<220>
   <223> Primer
<400> 14
   atagactaag cttaatgtgc tgaaagatat cttgtcac 38
<210> 15
   <211> 56
   <212> DNA
   <213> Artificial Sequence
<220>
   <223> Primer
<400> 15
   caattacata tgcatcacca tcaccatcac ttcagcctcc atgtaggtgc agatgc 56